# EUROPÄISCHE PATENTANMELDUNG

(11) **EP 2 497 829 A1**
(43) Veröffentlichungstag der Anmeldung: **12.09.2012**
(21) Anmeldenummer: 12075032.8
(22) Anmeldetag: 10.11.2005
(51) Int. Cl.: C12N 15/13, C12N 15/85, C12N 5/10, C07K 16/28, A61K 39/395, A61K 31/7088, A61K 35/12, A61P 37/02, A61P 37/04, A61P 37/06, G01N 33/53, G01N 33/577

(54) **Superagonistische anti-CD-28 Antikörper**

(30) Priorität: 11.11.2004 DE 102004055622; 11.05.2005 EP 05010264
(62) Teilanmeldung aus: 05802650.1
(71) Anmelder: TheraMAB LLC, Moscow 117246 (RU)
(72) Erfinder: Hanke, Thomas, 97209 Veitschöchheim (DE); Trischler, Martin, 97291 Thüngersheim (DE); Guntermann, Christine, 45659 Recklinghausen (DE)
(74) Vertreter: Jungblut, Bernhard Jakob

(57) **Zusammenfassung**

Die Erfindung betrifft eine Nukleinsäure oder mehrere Nukleinsäuren, kodierend ein Bindungsmolekül, das spezifisch ein menschliches CD28-Molekul bindet, umfassend (a) eine Nukleinsäuresequenz kodierend eine V_{H}-Region und eine Nukleinsäuresequenz kodierend eine V_{L}-Region umfassend CDRs in einem human Immunglobulin-Gerüst, wobei (i) die CDRs der VH-Region (CDR-H) die Aminosäuresequenzen der SEQ ID No. 2 oder 18 (CDR-H3), 4 oder 20 (CDR-H2) und 6 oder 22 (CDR-H1) umfassen oder kodiert werden durch die Nukleinsäuresequenzen der SEQ ID No. 1 oder 17 (CDR-H3), 3 oder 19 (CDR-H2) und 5 oder 21 (CDR-H1); und (ii) die CDRs der V_{L}-Region (CDR-L) die Aminosäuresequenzen der SEQ ID No. 8 oder 24 (CDR-L3), 10 oder 26 (CDR-L2) und 12 oder 28 (CDR-L1) umfassen oder kodiert werden durch die Nukleinsäuresequenzen der SEQ ID No. 7 oder 23 (CDR-L3), 9 oder 25 (CDR-L2) und 11 oder 27 (CDR-L1); und (b) eine Nukleinsauresequenz, die eine konstante Region eines humanen IgG1- oder IgG4-Antikörpers kodiert.

SEQUENCE LISTING

<110>  TeGenero AG

<120>  Superagonistische anti-CD28 Antikörper

<130>  L 1701 PCT

<160>  48

<170>  PatentIn version 3.1

<210>  1
<211>  33
<212>  DNA
<213>  artificial sequence

<220>
<223>  CDR

<400>  1
tcacactacg gcctcgactg gaacttcgat gtc                                    33

<210>  2
<211>  11
<212>  PRT
<213>  artificial sequence

<220>
<223>  CDR

<400>  2

Ser His Tyr Gly Leu Asp Trp Asn Phe Asp Val
1               5                   10

<210>  3
<211>  51
<212>  DNA
<213>  artificial sequence

<220>
<223>  CDR

<400>  3
tgtatttatc ctggaaatgt caatactaac tataatgaga agttcaagga c                51

<210>  4
<211>  17
<212>  PRT
<213>  artificial sequence

<220>
<223>  CDR

<400>  4

Cys Ile Tyr Pro Gly Asn Val Asn Thr Asn Tyr Asn Glu Lys Phe Lys
1               5                   10                  15

Asp

<210> 5
<211> 30
<212> DNA
<213> artificial sequence

<220>
<223> CDR

<400> 5
ggatacacct tcaccagcta ctatatacac 30

<210> 6
<211> 10
<212> PRT
<213> artificial sequence

<220>
<223> CDR

<400> 6

Gly Tyr Thr Phe Thr Ser Tyr Tyr Ile His
1               5                   10

<210> 7
<211> 27
<212> DNA
<213> artificial sequence

<220>
<223> CDR

<400> 7
caacagggtc aaacttatcc gtacacg 27

<210> 8
<211> 9
<212> PRT
<213> artificial sequence

<220>
<223> CDR

<400> 8

Gln Gln Gly Gln Thr Tyr Pro Tyr Thr
1               5

<210> 9
<211> 21
<212> DNA
<213> artificial sequence

<220>
<223> CDR

<400> 9
aaggcttcca acctgcacac a 21

<210> 10
<211> 7
<212> PRT
<213> artificial sequence

<220>
<223> CDR

<400> 10

Lys Ala Ser Asn Leu His Thr
1               5

<210> 11
<211> 33
<212> DNA
<213> artificial sequence

<220>
<223> CDR

<400> 11
catgccagtc aaaacattta tgtttggtta aac 33

<210> 12
<211> 11
<212> PRT
<213> artificial sequence

<220>
<223> CDR

<400> 12

His Ala Ser Gln Asn Ile Tyr Val Trp Leu Asn
1               5                   10

<210> 13
<211> 2178
<212> DNA
<213> artificial sequence

<220>
<223> HC

<400> 13
caggtgcagc tggtgcagtc tggggctgag gtgaagaagc ctggggcctc agtgaaggtc 60

tcctgcaagg cttctggata caccttcacc agctactata tacactgggt gcgacaggcc 120

cctggacaag ggcttgagtg gattggatgt atttatcctg gaaatgtcaa tactaactat 180

aatgagaagt tcaaggacag ggccaccctg accgtagaca cgtccatcag cacagcctac 240

atggagctga gcaggctgag atctgacgac acggccgtgt atttctgtac aagatcacac 300

tacggcctcg actggaactt cgatgtctgg ggccaaggga ccacggtcac cgtctcctca 360
ggtgagtcgt acgctagcaa gctttctggg gcaggccggg cctgactttg gctgggggca 420
gggagggggc taaggtgacg caggtggcgc cagccaggtg cacacccaat gcccatgagc 480
ccagacactg gaccctgcat ggaccatcgc ggatagacaa gaaccgaggg gcctctgcgc 540
cctgggccca gctctgtccc acaccgcggt cacatggcac cacctctctt gcagcttcca 600
ccaagggccc atccgtcttc cccctggcgc cctgctccag gagcacctcc gagagcacag 660
ccgccctggg ctgcctggtc aaggactact tccccgaacc ggtgacggtg tcgtggaact 720
caggcgccct gaccagcggc gtgcacacct tcccggctgt cctacagtcc tcaggactct 780
actccctcag cagcgtggtg accgtgccct ccagcagctt gggcacgaag acctacacct 840
gcaacgtaga tcacaagccc agcaacacca aggtggacaa gagagttggt gagaggccag 900
cacagggagg gagggtgtct gctggaagcc aggctcagcc ctcctgcctg gacgcacccc 960
ggctgtgcag ccccagccca gggcagcaag gcatgcccca tctgtctcct cacccggagg 1020
cctctgacca ccccactcat gctcagggag agggtcttct ggatttttcc accaggctcc 1080
gggcagccac aggctggatg cccctacccc aggccctgcg catacagggg caggtgctgc 1140
gctcagacct gccaagagcc atatccggga ggaccctgcc cctgacctaa gcccacccca 1200
aaggccaaac tctccactcc ctcagctcag acaccttctc tcctcccaga tctgagtaac 1260
tcccaatctt ctctctgcag agtccaaata tggtccccca tgcccatcat gcccaggtaa 1320
gccaacccag gcctcgccct ccagctcaag gcgggacagg tgccctagag tagcctgcat 1380
ccagggacag gccccagccg ggtgctgacg catccacctc catctcttcc tcagcacctg 1440
agttcctggg gggaccatca gtcttcctgt tccccccaaa acccaaggac actctcatga 1500
tctcccggac ccctgaggtc acgtgcgtgg tggtggacgt gagccaggaa gaccccgagg 1560
tccagttcaa ctggtacgtg gatggcgtgg aggtgcataa tgccaagaca aagccgcggg 1620
aggagcagtt caacagcacg taccgtgtgg tcagcgtcct caccgtcctg caccaggact 1680
ggctgaacgg caaggagtac aagtgcaagg tctccaacaa aggcctcccg tcctccatcg 1740
agaaaaccat ctccaaagcc aaaggtggga cccacggggt gcgagggcca catggacaga 1800
ggtcagctcg gcccaccctc tgccctggga gtgaccgctg tgccaacctc tgtccctaca 1860
gggcagcccc gagagccaca ggtgtacacc ctgcccccat cccaggagga gatgaccaag 1920
aaccaggtca gcctgacctg cctggtcaaa ggcttctacc ccagcgacat cgccgtggag 1980
tgggagagca atgggcagcc ggagaacaac tacaagacca cgcctcccgt gctggactcc 2040
gacggctcct tcttcctcta cagcaggcta accgtggaca agagcaggtg gcaggagggg 2100
aatgtcttct catgctccgt gatgcatgag gctctgcaca accactacac acagaagagc 2160
ctctccctgt ctctgggt 2178

<210>  14
<211>  446
<212>  PRT
<213>  artificial sequence

<220>
<223>  HC

<400>  14

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro

210                 215                 220

Pro Cys Pro Ser Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly
        435                 440                 445

<210>  15
<211>  1007
<212>  DNA
<213>  artificial sequence

<220>
<223>  LC

<400>  15
gacatccaga tgacccagtc tccatcctcc ctgtctgcat ctgtaggaga cagagtcacc      60

atcacttgcc atgccagtca aaacatttat gtttggttaa actggtatca gcagaaacca     120

gggaaagccc ctaagctcct gatctataag gcttccaacc tgcacacagg ggtcccatca     180

aggttcagtg gcagtggatc tgggacagat ttcactctca ccatcagcag tctgcaacct     240

gaagattttg caacttacta ctgtcaacag ggtcaaactt atccgtacac gttcggcgga     300

gggaccaagg tggagatcaa acgtgagtcg tacgctagca agcttgatat cgaattctaa     360

actctgaggg ggtcggatga cgtggccatt ctttgcctaa agcattgagt ttactgcaag     420

gtcagaaaag catgcaaagc cctcagaatg gctgcaaaga gctccaacaa aacaatttag     480

aactttatta aggaataggg ggaagctagg aagaaactca aaacatcaag attttaaata     540

cgcttcttgg tctccttgct ataattatct gggataagca tgctgttttc tgtctgtccc     600

taacatgccc tgtgattatc cgcaaacaac acacccaagg gcagaacttt gttacttaaa     660

caccatcctg tttgcttctt tcctcaggaa ctgtggctgc accatctgtc ttcatcttcc     720

cgccatctga tgagcagttg aaatctggaa ctgcctctgt tgtgtgcctg ctgaataact     780

tctatcccag agaggccaaa gtacagtgga aggtggataa cgccctccaa tcgggtaact     840

cccaggagag tgtcacagag caggacagca aggacagcac ctacagcctc agcagcaccc     900

tgacgctgag caaagcagac tacgagaaac acaaagtcta cgcctgcgaa gtcacccatc     960

agggcctgag ctcgcccgtc acaaagagct tcaacagggg agagtgt                  1007

<210>  16
<211>  214
<212>  PRT
<213>  artificial sequence

<220>
<223>  LC

<400>  16

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asn Ile Tyr Val Trp
            20                  25                  30

Leu Asn Trp Tyr Gln Gln Lys Pro Gly Lys Ala Pro Lys Leu Leu Ile
        35                  40                  45

Tyr Lys Ala Ser Asn Leu His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Gln Thr Tyr Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  17
<211>  33
<212>  DNA
<213>  artificial sequence

<220>
<223>  CDR

<400>  17
tcacactacg gcctcgactg gaacttcgat gtc                                    33

<210>  18
<211>  11
<212>  PRT
<213>  artificial sequence

<220>
<223>  CDR

<400>  18

Ser His Tyr Gly Leu Asp Trp Asn Phe Asp Val

1               5                   10


<210>  19
<211>  51
<212>  DNA
<213>  artificial sequence

<220>
<223>  CDR

<400>  19
tgtatttatc ctggaaatgt caatactaac tataatgaga agttcaagga c                51


<210>  20
<211>  17
<212>  PRT
<213>  artificial sequence

<220>
<223>  CDR

<400>  20

Cys Ile Tyr Pro Gly Asn Val Asn Thr Asn Tyr Asn Glu Lys Phe Lys
1               5                   10                  15


Asp


<210>  21
<211>  30
<212>  DNA
<213>  artificial sequence

<220>
<223>  CDR

<400>  21
ggatacacct tcaccagcta ctatatacac                                        30


<210>  22
<211>  10
<212>  PRT
<213>  artificial sequence

<220>
<223>  CDR

<400>  22

Gly Tyr Thr Phe Thr Ser Tyr Tyr Ile His
1               5                   10


<210>  23
<211>  27
<212>  DNA
<213>  artificial sequence

<220>
<223>  CDR

<400>  23
caacagggtc aaacttatcc gtacacg                                          27


<210>  24
<211>  9
<212>  PRT
<213>  artificial sequence

<220>
<223>  CDR

<400>  24

Gln Gln Gly Gln Thr Tyr Pro Tyr Thr
1               5


<210>  25
<211>  21
<212>  DNA
<213>  artificial sequence

<220>
<223>  CDR

<400>  25
aaggcttcca acctgcacac a                                                21


<210>  26
<211>  7
<212>  PRT
<213>  artificial sequence

<220>
<223>  CDR

<400>  26

Lys Ala Ser Asn Leu His Thr
1               5


<210>  27
<211>  33
<212>  DNA
<213>  artificial sequence

<220>
<223>  CDR

<400>  27
catgccagtc aaaacattta tgtttggtta aac                                   33


<210>  28
<211>  11
<212>  PRT
<213>  artificial sequence

<220>
<223>  CDR

<400>  28

His Ala Ser Gln Asn Ile Tyr Val Trp Leu Asn
1               5                   10


<210>  29
<211>  2184
<212>  DNA
<213>  artificial sequence

<220>
<223>  HC

<400>  29
caggtgcagc tggtgcagtc tggggctgag gtgaagaagc ctggggcctc agtgaaggtc      60

tcctgcaagg cttctggata caccttcacc agctactata tacactgggt gcgacaggcc     120

cctggacaag ggcttgagtg gattggatgt atttatcctg gaaatgtcaa tactaactat     180

aatgagaagt tcaaggacag ggccaccctg accgtagaca cgtccatcag cacagcctac     240

atggagctga gcaggctgag atctgacgac acggccgtgt atttctgtac aagatcacac     300

tacggcctcg actggaactt cgatgtctgg ggccaaggga ccacggtcac cgtctcctca     360

ggtgagtcgt acgctagcaa gctttctggg gcaggccagg cctgaccttg gctttggggc     420

agggaggggg ctaaggtgag gcaggtggcg ccagccaggt gcacacccaa tgcccatgag     480

cccagacact ggacgctgaa cctcgcggac agttaagaac ccaggggcct ctgcgccctg     540

ggcccagctc tgtcccacac cgcggtcaca tggcaccacc tctcttgcag cctccaccaa     600

gggcccatcg gtcttccccc tggcaccctc ctccaagagc acctctgggg gcacagcggc     660

cctgggctgc ctggtcaagg actacttccc cgaaccggtg acggtgtcgt ggaactcagg     720

cgccctgacc agcggcgtgc acaccttccc ggctgtccta cagtcctcag gactctactc     780

cctcagcagc gtggtgaccg tgccctccag cagcttgggc acccagacct acatctgcaa     840

cgtgaatcac aagcccagca acaccaaggt ggacaagaaa gttggtgaga ggccagcaca     900

gggagggagg gtgtctgctg gaagccaggc tcagcgctcc tgcctggacg catcccggct     960

atgcagcccc agtccagggc agcaaggcag gccccgtctg cctcttcacc cggaggcctc    1020

tgcccgcccc actcatgctc agggagaggg tcttctggct ttttcccagg ctctgggcag    1080

gcacaggcta ggtgccccta acccaggccc tgcacacaaa ggggcaggtg ctgggctcag    1140

acctgccaag agccatatcc gggaggaccc tgcccctgac ctaagcccac cccaaaggcc    1200

aaactctcca ctccctcagc tcggacacct tctctcctcc cagattccag taactcccaa    1260

tcttctctct gcagagccca aatcttgtga caaaactcac acatgcccac cgtgcccagg    1320

taagccagcc caggcctcgc cctccagctc aaggcgggac aggtgcccta gagtagcctg    1380

catccaggga caggccccag ccgggtgctg acacgtccac ctccatctct tcctcagcac 1440

ctgaactcct ggggggaccg tcagtcttcc tcttcccccc aaaacccaag gacaccctca 1500

tgatctcccg gacccctgag gtcacatgcg tggtggtgga cgtgagccac gaagaccctg 1560

aggtcaagtt caactggtac gtggacggcg tggaggtgca taatgccaag acaaagccgc 1620

gggaggagca gtacaacagc acgtaccggg tggtcagcgt cctcaccgtc ctgcaccagg 1680

actggctgaa tggcaaggag tacaagtgca aggtctccaa caaagccctc ccagccccca 1740

tcgagaaaac catctccaaa gccaaaggtg ggacccgtgg ggtgcgaggg ccacatggac 1800

agaggccggc tcggcccacc ctctgccctg agagtgaccg ctgtaccaac ctctgtccct 1860

acagggcagc cccgagaacc acaggtgtac accctgcccc catcccggga tgagctgacc 1920

aagaaccagg tcagcctgac ctgcctggtc aaaggcttct atcccagcga catcgccgtg 1980

gagtgggaga gcaatgggca gccggagaac aactacaaga ccacgcctcc cgtgctggac 2040

tccgacggct ccttcttcct ctacagcaag ctcaccgtgg acaagagcag gtggcagcag 2100

gggaacgtct tctcatgctc cgtgatgcat gaggctctgc acaaccacta cacgcagaag 2160

agcctctccc tgtctccggg taaa 2184

<210> 30
<211> 450
<212> PRT
<213> artificial sequence

<220>
<223> HC

<400> 30

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln

            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu

355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  31
<211>  1007
<212>  DNA
<213>  artificial sequence

<220>
<223>  LC

<400>  31
gacatccaga tgacccagtc tccatcctcc ctgtctgcat ctgtaggaga cagagtcacc      60

atcacttgcc atgccagtca aaacatttat gtttggttaa actggtatca gcagaaacca     120

gggaaagccc ctaagctcct gatctataag gcttccaacc tgcacacagg ggtcccatca     180

aggttcagtg gcagtggatc tgggacagat ttcactctca ccatcagcag tctgcaacct     240

gaagattttg caacttacta ctgtcaacag ggtcaaactt atccgtacac gttcggcgga     300

gggaccaagg tggagatcaa acgtgagtcg tacgctagca agcttgatat cgaattctaa     360

actctgaggg ggtcggatga cgtggccatt ctttgcctaa agcattgagt ttactgcaag     420

gtcagaaaag catgcaaagc cctcagaatg gctgcaaaga gctccaacaa aacaatttag     480

aactttatta aggaataggg ggaagctagg aagaaactca aaacatcaag attttaaata     540

cgcttcttgg tctccttgct ataattatct gggataagca tgctgttttc tgtctgtccc     600

taacatgccc tgtgattatc cgcaaacaac acacccaagg gcagaacttt gttacttaaa     660

caccatcctg tttgcttctt tcctcaggaa ctgtggctgc accatctgtc ttcatcttcc     720

cgccatctga tgagcagttg aaatctggaa ctgcctctgt tgtgtgcctg ctgaataact     780

tctatcccag agaggccaaa gtacagtgga aggtggataa cgccctccaa tcgggtaact     840

cccaggagag tgtcacagag caggacagca aggacagcac ctacagcctc agcagcaccc 900

tgacgctgag caaagcagac tacgagaaac acaaagtcta cgcctgcgaa gtcacccatc 960

agggcctgag ctcgcccgtc acaaagagct tcaacagggg agagtgt 1007

<210> 32
<211> 214
<212> PRT
<213> artificial sequence

<220>
<223> LC

<400> 32

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asn Ile Tyr Val Trp
            20                  25                  30

Leu Asn Trp Tyr Gln Gln Lys Pro Gly Lys Ala Pro Lys Leu Leu Ile
        35                  40                  45

Tyr Lys Ala Ser Asn Leu His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Gln Thr Tyr Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  33
<211>  360
<212>  DNA
<213>  artificial sequence

<220>
<223>  VHC

<400>  33
caggtgcagc tggtgcagtc tggggctgag gtgaagaagc ctggggcctc agtgaaggtc      60

tcctgcaagg cttctggata caccttcacc agctactata tacactgggt gcgacaggcc     120

cctggacaag ggcttgagtg gattggatgt atttatcctg gaaatgtcaa tactaactat     180

aatgagaagt tcaaggacag ggccaccctg accgtagaca cgtccatcag cacagcctac     240

atggagctga gcaggctgag atctgacgac acggccgtgt atttctgtac aagatcacac     300

tacggcctcg actggaactt cgatgtctgg ggccaaggga ccacggtcac cgtctcctca     360

<210>  34
<211>  120
<212>  PRT
<213>  artificial sequence

<220>
<223>  VHC

<400>  34

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
        100                 105                 110

Gly Thr Thr Val Thr Val Ser Ser
        115                 120

<210> 35
<211> 321
<212> DNA
<213> artificial sequence

<220>
<223> VLC

<400> 35
gacatccaga tgacccagtc tccatcctcc ctgtctgcat ctgtaggaga cagagtcacc      60

atcacttgcc atgccagtca aaacatttat gtttggttaa actggtatca gcagaaacca     120

gggaaagccc ctaagctcct gatctataag gcttccaacc tgcacacagg ggtcccatca     180

aggttcagtg gcagtggatc tgggacagat ttcactctca ccatcagcag tctgcaacct     240

gaagattttg caacttacta ctgtcaacag ggtcaaactt atccgtacac gttcggcgga     300

gggaccaagg tggagatcaa a                                               321

<210> 36
<211> 107
<212> PRT
<213> artificial sequence

<220>
<223> VLC

<400> 36

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asn Ile Tyr Val Trp
            20                  25                  30

Leu Asn Trp Tyr Gln Gln Lys Pro Gly Lys Ala Pro Lys Leu Leu Ile
        35                  40                  45

Tyr Lys Ala Ser Asn Leu His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Gln Thr Tyr Pro Tyr
                85                  90                  95

Thr Phe Gly Gly Gly Thr Lys Val Glu Ile Lys
            100                 105

<210> 37
<211> 360
<212> DNA
<213> artificial sequence

<220>
<223> VHC

<400> 37
caggtgcagc tggtgcagtc tggggctgag gtgaagaagc ctggggcctc agtgaaggtc 60

tcctgcaagg cttctggata caccttcacc agctactata tacactgggt gcgacaggcc 120

cctggacaag ggcttgagtg gattggatgt atttatcctg gaaatgtcaa tactaactat 180

aatgagaagt tcaaggacag ggccaccctg accgtagaca cgtccatcag cacagcctac 240

atggagctga gcaggctgag atctgacgac acggccgtgt atttctgtac aagatcacac 300

tacggcctcg actggaactt cgatgtctgg ggccaaggga ccacggtcac cgtctcctca 360

<210> 38
<211> 120
<212> PRT
<213> artificial sequence

<220>
<223> VHC

<400> 38

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser
        115                 120

<210>  39
<211>  321
<212>  DNA
<213>  artificial sequence

<220>
<223>  VLC

<400>  39
gacatccaga tgacccagtc tccatcctcc ctgtctgcat ctgtaggaga cagagtcacc      60

atcacttgcc atgccagtca aaacatttat gtttggttaa actggtatca gcagaaacca     120

gggaaagccc ctaagctcct gatctataag gcttccaacc tgcacacagg ggtcccatca     180

aggttcagtg gcagtggatc tgggacagat ttcactctca ccatcagcag tctgcaacct     240

gaagattttg caacttacta ctgtcaacag ggtcaaactt atccgtacac gttcggcgga     300

gggaccaagg tggagatcaa a                                               321

<210>  40
<211>  107
<212>  PRT
<213>  artificial sequence

<220>
<223>  VLC

<400>  40

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asn Ile Tyr Val Trp
            20                  25                  30

Leu Asn Trp Tyr Gln Gln Lys Pro Gly Lys Ala Pro Lys Leu Leu Ile
        35                  40                  45

Tyr Lys Ala Ser Asn Leu His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Gln Thr Tyr Pro Tyr
                85                  90                  95

Thr Phe Gly Gly Gly Thr Lys Val Glu Ile Lys
            100                 105

<210> 41
<211> 2551
<212> DNA
<213> artificial sequence

<220>
<223> HC

<400> 41
ggtaccgggc cgacctcacc atgggatgga gctgtatcat cctcttcttg gtagcaacag 60

ctacaggtaa ggggctcaca gtagcaggct tgaggtctgg acatatatat gggtgacaat 120

gacatccact ttgcctttct ctccacaggt gtgcattccc aggtgcagct ggtgcagtct 180

ggggctgagg tgaagaagcc tggggcctca gtgaaggtct cctgcaaggc ttctggatac 240

accttcacca gctactatat acactgggtg cgacaggccc ctggacaagg gcttgagtgg 300

attggatgta tttatcctgg aaatgtcaat actaactata atgagaagtt caaggacagg 360

gccaccctga ccgtagacac gtccatcagc acagcctaca tggagctgag caggctgaga 420

tctgacgaca cggccgtgta tttctgtaca agatcacact acggcctcga ctggaacttc 480

gatgtctggg gccaagggac cacggtcacc gtctcctcag gtgagtcgta cgctagcaag 540

ctttctgggg caggccgggc ctgactttgg ctgggggcag ggagggggct aaggtgacgc 600

aggtggcgcc agccaggtgc acacccaatg cccatgagcc cagacactgg accctgcatg 660

gaccatcgcg gatagacaag aaccgagggg cctctgcgcc ctgggcccag ctctgtccca 720

caccgcggtc acatggcacc acctctcttg cagcttccac caagggccca tccgtcttcc 780

ccctggcgcc ctgctccagg agcacctccg agagcacagc cgccctgggc tgcctggtca 840

aggactactt ccccgaaccg gtcacggtgt cgtggaactc aggcgccctg accagcggcg 900

tgcacacctt cccggctgtc ctacagtcct caggactcta ctccctcagc agcgtggtga 960

ccgtgccctc cagcagcttg ggcacgaaga cctacacctg caacgtagat cacaagccca 1020

gcaacaccaa ggtggacaag agagttggtg agaggccagc acagggaggg agggtgtctg 1080

ctggaagcca ggctcagccc tcctgcctgg acgcaccccg gctgtgcagc cccagcccag 1140

ggcagcaagg catgccccat ctgtctcctc acccggaggc ctctgaccac cccactcatg 1200

ctcagggaga gggtcttctg gatttttcca ccaggctccg ggcagccaca ggctggatgc 1260

ccctacccca ggccctgcgc atacaggggc aggtgctgcg ctcagacctg ccaagagcca 1320

tatccgggag gaccctgccc ctgacctaag cccaccccaa aggccaaact ctccactccc 1380

tcagctcaga caccttctct cctcccagat ctgagtaact cccaatcttc tctctgcaga 1440

gtccaaatat ggtcccccat gcccatcatg cccaggtaag ccaacccagg cctcgccctc 1500

cagctcaagg cgggacaggt gccctagagt agcctgcatc cagggacagg ccccagccgg 1560

gtgctgacgc atccacctcc atctcttcct cagcacctga gttcctgggg ggaccatcag 1620

tcttcctgtt cccccaaaa cccaaggaca ctctcatgat ctcccggacc cctgaggtca 1680

cgtgcgtggt ggtggacgtg agccaggaag accccgaggt ccagttcaac tggtacgtgg 1740

atggcgtgga ggtgcataat gccaagacaa agccgcggga ggagcagttc aacagcacgt 1800

accgtgtggt cagcgtcctc accgtcctgc accaggactg gctgaacggc aaggagtaca 1860

agtgcaaggt ctccaacaaa ggcctcccgt cctccatcga gaaaaccatc tccaaagcca 1920

aaggtgggac ccacggggtg cgagggccac atggacagag gtcagctcgg cccaccctct 1980

gccctgggag tgaccgctgt gccaacctct gtccctacag ggcagccccg agagccacag 2040

gtgtacaccc tgcccccatc ccaggaggag atgaccaaga accaggtcag cctgacctgc 2100

ctggtcaaag gcttctaccc cagcgacatc gccgtggagt gggagagcaa tgggcagccg 2160

gagaacaact acaagaccac gcctcccgtg ctggactccg acggctcctt cttcctctac 2220

agcaggctaa ccgtggacaa gagcaggtgg caggagggga atgtcttctc atgctccgtg 2280

atgcatgagg ctctgcacaa ccactacaca cagaagagcc tctccctgtc tctgggtaaa 2340

tgagtgccag ggccggcaag cccccgctcc ccgggctctc ggggtcgcgc gaggatgctt 2400

ggcacgtacc ccgtctacat acttcccagg cacccagcat ggaaataaag cacccaccac 2460

tgccctgggc ccctgtgaga ctgtgatggt tctttccacg ggtcaggccg agtctgaggc 2520

ctgagtgaca tgagggaggc agagcggatc c 2551

<210> 42
<211> 466
<212> PRT
<213> artificial sequence

<220>
<223> HC

<400> 42

Met Gly Trp Ser Cys Ile Ile Leu Phe Leu Val Ala Thr Ala Thr Gly
1               5                   10                  15

Val His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Tyr Ile His Trp Val Arg Gln Ala Pro Gly Gln Gly Leu
    50                  55                  60

Glu Trp Ile Gly Cys Ile Tyr Pro Gly Asn Val Asn Thr Asn Tyr Asn
65                  70                  75                  80

Glu Lys Phe Lys Asp Arg Ala Thr Leu Thr Val Asp Thr Ser Ile Ser

                85                  90                  95

Thr Ala Tyr Met Glu Leu Ser Arg Leu Arg Ser Asp Asp Thr Ala Val
            100                 105                 110

Tyr Phe Cys Thr Arg Ser His Tyr Gly Leu Asp Trp Asn Phe Asp Val
        115                 120                 125

Trp Gly Gln Gly Thr Thr Val Thr Val Ser Ser Ala Ser Thr Lys Gly
    130                 135                 140

Pro Ser Val Phe Pro Leu Ala Pro Cys Ser Arg Ser Thr Ser Glu Ser
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Lys Thr Tyr Thr Cys Asn Val
    210                 215                 220

Asp His Lys Pro Ser Asn Thr Lys Val Asp Lys Arg Val Glu Ser Lys
225                 230                 235                 240

Tyr Gly Pro Pro Cys Pro Ser Cys Pro Ala Pro Glu Phe Leu Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser Gln Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Tyr Arg
305                 310                 315                 320

Val Val Ser Val Leu Thr Val Leu His Gln Asp Trp Leu Asn Gly Lys
                325                 330                 335

Glu Tyr Lys Cys Lys Val Ser Asn Lys Gly Leu Pro Ser Ser Ile Glu

340                 345                 350

Lys Thr Ile Ser Lys Ala Lys Gly Gln Pro Arg Glu Pro Gln Val Tyr
        355                 360                 365

Thr Leu Pro Pro Ser Gln Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Val
                405                 410                 415

Leu Asp Ser Asp Gly Ser Phe Phe Leu Tyr Ser Arg Leu Thr Val Asp
            420                 425                 430

Lys Ser Arg Trp Gln Glu Gly Asn Val Phe Ser Cys Ser Val Met His
        435                 440                 445

Glu Ala Leu His Asn His Tyr Thr Gln Lys Ser Leu Ser Leu Ser Leu
    450                 455                 460

Gly Lys
465

<210>  43
<211>  1721
<212>  DNA
<213>  artificial sequence

<220>
<223>  LC

<400>  43
   ggtaccgggc cgacctcacc atgggatgga gctgtatcat cctcttcttg gtagcaacag      60
   ctacaggtaa ggggctcaca gtagcaggct tgaggtctgg acatatatat gggtgacaat     120
   gacatccact ttgcctttct ctccacaggt gtgcattccg acatccagat gacccagtct     180
   ccatcctccc tgtctgcatc tgtaggagac agagtcacca tcacttgcca tgccagtcaa     240
   aacatttatg tttggttaaa ctggtatcag cagaaaccag ggaaagcccc taagctcctg     300
   atctataagg cttccaacct gcacacaggg gtcccatcaa ggttcagtgg cagtggatct     360
   gggacagatt tcactctcac catcagcagt ctgcaacctg aagattttgc aacttactac     420
   tgtcaacagg gtcaaactta tccgtacacg ttcggcggag ggaccaaggt ggagatcaaa     480
   cgtgagtcgt acgctagcaa gcttgatatc gaattctaaa ctctgagggg gtcggatgac     540
   gtggccattc tttgcctaaa gcattgagtt tactgcaagg tcagaaaagc atgcaaagcc     600
   ctcagaatgg ctgcaaagag ctccaacaaa acaatttaga actttattaa ggaatagggg 660
   gaagctagga agaaactcaa aacatcaaga ttttaaatac gcttcttggt ctccttgcta 720
   taattatctg ggataagcat gctgttttct gtctgtccct aacatgccct gtgattatcc 780
   gcaaacaaca cacccaaggg cagaactttg ttacttaaac accatcctgt ttgcttcttt 840
   cctcaggaac tgtggctgca ccatctgtct tcatcttccc gccatctgat gagcagttga 900
   aatctggaac tgcctctgtt gtgtgcctgc tgaataactt ctatcccaga gaggccaaag 960
   tacagtggaa ggtggataac gccctccaat cgggtaactc ccaggagagt gtcacagagc 1020
   aggacagcaa ggacagcacc tacagcctca gcagcaccct gacgctgagc aaagcagact 1080
   acgagaaaca caaagtctac gcctgcgaag tcacccatca gggcctgagc tcgcccgtca 1140
   caaagagctt caacagggga gagtgttaga gggagaagtg cccccacctg ctcctcagtt 1200
   ccagcctgac cccctcccat cctttggcct ctgacccttt ttccacaggg gacctacccc 1260
   tattgcggtc ctccagctca tctttcacct cacccccctc ctcctccttg gctttaatta 1320
   tgctaatgtt ggaggagaat gaataaataa agtgaatctt tgcacctgtg gtttctctct 1380
   ttcctcattt aataattatt atctgttgtt ttaccaacta ctcaatttct cttataaggg 1440
   actaaatatg tagtcatcct aaggcgcata accatttata aaaatcatcc ttcattctat 1500
   tttaccctat catcctctgc aagacagtcc tccctcaaac ccacaagcct tctgtcctca 1560
   cagtcccctg ggccatggta ggagagactt gcttccttgt tttcccctcc tcagcaagcc 1620
   ctcatagtcc tttttaaggg tgacaggtct tacagtcata tatcctttga ttcaattccc 1680
   tgagaatcaa ccaaagcaaa ttcctgcagc ccgggggatc c 1721

<210> 44
<211> 233
<212> PRT
<213> artificial sequence

<220>
<223> LC

<400> 44

Met Gly Trp Ser Cys Ile Ile Leu Phe Leu Val Ala Thr Ala Thr Gly
1               5                   10                  15

Val His Ser Asp Ile Gln Met Thr Gln Ser Pro Ser Ser Leu Ser Ala
            20                  25                  30

Ser Val Gly Asp Arg Val Thr Ile Thr Cys His Ala Ser Gln Asn Ile
        35                  40                  45

Tyr Val Trp Leu Asn Trp Tyr Gln Gln Lys Pro Gly Lys Ala Pro Lys
    50                  55                  60

Leu Leu Ile Tyr Lys Ala Ser Asn Leu His Thr Gly Val Pro Ser Arg
65                  70                  75                  80

Phe Ser Gly Ser Gly Ser Gly Thr Asp Phe Thr Leu Thr Ile Ser Ser
                85                  90                  95

Leu Gln Pro Glu Asp Phe Ala Thr Tyr Tyr Cys Gln Gln Gly Gln Thr
            100                 105                 110

Tyr Pro Tyr Thr Phe Gly Gly Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

<210>  45
<211>  2553
<212>  DNA
<213>  artificial sequence

<220>
<223>  HC

<400>  45
ggtaccgggc cgacctcacc atgggatgga gctgtatcat cctcttcttg gtagcaacag      60

ctacaggtaa ggggctcaca gtagcaggct tgaggtctgg acatatatat gggtgacaat     120

gacatccact ttgcctttct ctccacaggt gtgcattccc aggtgcagct ggtgcagtct     180

ggggctgagg tgaagaagcc tggggcctca gtgaaggtct cctgcaaggc ttctggatac     240

accttcacca gctactatat acactgggtg cgacaggccc ctggacaagg gcttgagtgg     300

attggatgta tttatcctgg aaatgtcaat actaactata atgagaagtt caaggacagg 360
gccaccctga ccgtagacac gtccatcagc acagcctaca tggagctgag caggctgaga 420
tctgacgaca cggccgtgta tttctgtaca agatcacact acggcctcga ctggaacttc 480
gatgtctggg gccaagggac cacggtcacc gtctcctcag gtgagtcgta cgctagcaag 540
ctttctgggg caggccaggc ctgaccttgg ctttggggca gggagggggc taaggtgagg 600
caggtggcgc cagccaggtg cacacccaat gcccatgagc ccagacactg gacgctgaac 660
ctcgcggaca gttaagaacc caggggcctc tgcgccctgg gcccagctct gtcccacacc 720
gcggtcacat ggcaccacct ctcttgcagc ctccaccaag ggcccatcgg tcttccccct 780
ggcaccctcc tccaagagca cctctggggg cacagcggcc ctgggctgcc tggtcaagga 840
ctacttcccc gaaccggtga cggtgtcgtg gaactcaggc gccctgacca gcggcgtgca 900
caccttcccg gctgtcctac agtcctcagg actctactcc ctcagcagcg tggtgaccgt 960
gccctccagc agcttgggca cccagaccta catctgcaac gtgaatcaca agcccagcaa 1020
caccaaggtg gacaagaaag ttggtgagag gccagcacag ggagggaggg tgtctgctgg 1080
aagccaggct cagcgctcct gcctggacgc atcccggcta tgcagcccca gtccagggca 1140
gcaaggcagg ccccgtctgc ctcttcaccc ggaggcctct gcccgcccca ctcatgctca 1200
gggagagggt cttctggctt tttcccaggc tctgggcagg cacaggctag gtgcccctaa 1260
cccaggccct gcacacaaag gggcaggtgc tgggctcaga cctgccaaga gccatatccg 1320
ggaggaccct gcccctgacc taagcccacc ccaaaggcca aactctccac tccctcagct 1380
cggacacctt ctctcctccc agattccagt aactcccaat cttctctctg cagagcccaa 1440
atcttgtgac aaaactcaca catgcccacc gtgcccaggt aagccagccc aggcctcgcc 1500
ctccagctca aggcgggaca ggtgccctag agtagcctgc atccagggac aggccccagc 1560
cgggtgctga cacgtccacc tccatctctt cctcagcacc tgaactcctg gggggaccgt 1620
cagtcttcct cttcccccca aaacccaagg acaccctcat gatctcccgg acccctgagg 1680
tcacatgcgt ggtggtggac gtgagccacg aagaccctga ggtcaagttc aactggtacg 1740
tggacggcgt ggaggtgcat aatgccaaga caaagccgcg ggaggagcag tacaacagca 1800
cgtaccgggt ggtcagcgtc ctcaccgtcc tgcaccagga ctggctgaat ggcaaggagt 1860
acaagtgcaa ggtctccaac aaagccctcc cagcccccat cgagaaaacc atctccaaag 1920
ccaaaggtgg gacccgtggg gtgcgagggc cacatggaca gaggccggct cggcccaccc 1980
tctgccctga gagtgaccgc tgtaccaacc tctgtcccta cagggcagcc ccgagaacca 2040
caggtgtaca ccctgccccc atcccgggat gagctgacca agaaccaggt cagcctgacc 2100
tgcctggtca aaggcttcta tcccagcgac atcgccgtgg agtgggagag caatgggcag 2160
ccggagaaca actacaagac cacgcctccc gtgctggact ccgacggctc cttcttcctc 2220

tacagcaagc tcaccgtgga caagagcagg tggcagcagg ggaacgtctt ctcatgctcc 2280

gtgatgcatg aggctctgca caaccactac acgcagaaga gcctctccct gtctccgggt 2340

aaatgagtgc gacggccggc aagcccccgc tccccgggct ctcgcggtcg cacgaggatg 2400

cttggcacgt accccctgta catacttccc gggcgcccag catggaaata aagcacccag 2460

cgctgccctg ggcccctgcg agactgtgat ggttctttcc acgggtcagg ccgagtctga 2520

ggcctgagtg gcatgaggga ggcagagcgg gtc 2553

<210> 46
<211> 469
<212> PRT
<213> artificial sequence

<220>
<223> HC

<400> 46

Met Gly Trp Ser Cys Ile Ile Leu Phe Leu Val Ala Thr Ala Thr Gly
1               5                   10                  15

Val His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Tyr Ile His Trp Val Arg Gln Ala Pro Gly Gln Gly Leu
    50                  55                  60

Glu Trp Ile Gly Cys Ile Tyr Pro Gly Asn Val Asn Thr Asn Tyr Asn
65                  70                  75                  80

Glu Lys Phe Lys Asp Arg Ala Thr Leu Thr Val Asp Thr Ser Ile Ser
                85                  90                  95

Thr Ala Tyr Met Glu Leu Ser Arg Leu Arg Ser Asp Asp Thr Ala Val
            100                 105                 110

Tyr Phe Cys Thr Arg Ser His Tyr Gly Leu Asp Trp Asn Phe Asp Val
        115                 120                 125

Trp Gly Gln Gly Thr Thr Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val

                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu

420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210>  47
<211>  1721
<212>  DNA
<213>  artificial sequence

<220>
<223>  LC

<400>  47
ggtaccgggc cgacctcacc atgggatgga gctgtatcat cctcttcttg gtagcaacag      60

ctacaggtaa ggggctcaca gtagcaggct tgaggtctgg acatatatat gggtgacaat     120

gacatccact ttgcctttct ctccacaggt gtgcattccg acatccagat gacccagtct     180

ccatcctccc tgtctgcatc tgtaggagac agagtcacca tcacttgcca tgccagtcaa     240

aacatttatg tttggttaaa ctggtatcag cagaaaccag ggaaagcccc taagctcctg     300

atctataagg cttccaacct gcacacaggg gtcccatcaa ggttcagtgg cagtggatct     360

gggacagatt tcactctcac catcagcagt ctgcaacctg aagattttgc aacttactac     420

tgtcaacagg gtcaaactta tccgtacacg ttcggcggag ggaccaaggt ggagatcaaa     480

cgtgagtcgt acgctagcaa gcttgatatc gaattctaaa ctctgagggg gtcggatgac     540

gtggccattc tttgcctaaa gcattgagtt tactgcaagg tcagaaaagc atgcaaagcc     600

ctcagaatgg ctgcaaagag ctccaacaaa acaatttaga actttattaa ggaatagggg     660

gaagctagga agaaactcaa aacatcaaga ttttaaatac gcttcttggt ctccttgcta     720

taattatctg ggataagcat gctgttttct gtctgtccct aacatgccct gtgattatcc     780

gcaaacaaca cacccaaggg cagaactttg ttacttaaac accatcctgt ttgcttcttt     840

cctcaggaac tgtggctgca ccatctgtct tcatcttccc gccatctgat gagcagttga     900

aatctggaac tgcctctgtt gtgtgcctgc tgaataactt ctatcccaga gaggccaaag     960

tacagtggaa ggtggataac gccctccaat cgggtaactc ccaggagagt gtcacagagc    1020

aggacagcaa ggacagcacc tacagcctca gcagcaccct gacgctgagc aaagcagact    1080

acgagaaaca caaagtctac gcctgcgaag tcacccatca gggcctgagc tcgcccgtca    1140

caaagagctt caacagggga gagtgttaga gggagaagtg cccccacctg ctcctcagtt    1200

ccagcctgac cccctcccat cctttggcct ctgacccttt ttccacaggg gacctacccc 1260

tattgcggtc ctccagctca tctttcacct caccccctc ctcctccttg gctttaatta 1320

tgctaatgtt ggaggagaat gaataaataa agtgaatctt tgcacctgtg gtttctctct 1380

ttcctcattt aataattatt atctgttgtt ttaccaacta ctcaatttct cttataaggg 1440

actaaatatg tagtcatcct aaggcgcata accatttata aaaatcatcc ttcattctat 1500

tttaccctat catcctctgc aagacagtcc tccctcaaac ccacaagcct tctgtcctca 1560

cagtcccctg ggccatggta ggagagactt gcttccttgt tttcccctcc tcagcaagcc 1620

ctcatagtcc tttttaaggg tgacaggtct tacagtcata tatcctttga ttcaattccc 1680

tgagaatcaa ccaaagcaaa ttcctgcagc ccgggggatc c 1721

<210> 48
<211> 233
<212> PRT
<213> artificial sequence

<220>
<223> LC

<400> 48

Met Gly Trp Ser Cys Ile Ile Leu Phe Leu Val Ala Thr Ala Thr Gly
1               5                   10                  15

Val His Ser Asp Ile Gln Met Thr Gln Ser Pro Ser Ser Leu Ser Ala
            20                  25                  30

Ser Val Gly Asp Arg Val Thr Ile Thr Cys His Ala Ser Gln Asn Ile
        35                  40                  45

Tyr Val Trp Leu Asn Trp Tyr Gln Gln Lys Pro Gly Lys Ala Pro Lys
    50                  55                  60

Leu Leu Ile Tyr Lys Ala Ser Asn Leu His Thr Gly Val Pro Ser Arg
65                  70                  75                  80

Phe Ser Gly Ser Gly Ser Gly Thr Asp Phe Thr Leu Thr Ile Ser Ser
                85                  90                  95

Leu Gln Pro Glu Asp Phe Ala Thr Tyr Tyr Cys Gln Gln Gly Gln Thr
            100                 105                 110

Tyr Pro Tyr Thr Phe Gly Gly Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

## Beschreibung

Die Erfindung betrifft eine Nukleinsäure oder mehrere Nukleinsäuren, kodierend ein Bindungsmolekül, das spezifisch ein menschliches CD28-Molekül bindet, umfassend
(a) eine Nukleinsäuresequenz kodierend eine V_{H}-Region und eine Nukleinsäuresequenz kodierend eine V_{L}-Region umfassend CDRs in einem humanen Immunglobulin-Gerüst, wobei
   (i) die CDRs der V_{H}-Region (CDR-H) die Aminosäuresequenzen der SEQ ID NO: 2 oder 18 (CDR-H3), 4 oder 20 (CDR-H2) und 6 oder 22 (CDR-H1) umfassen oder
      kodiert werden durch die Nukleinsäuresequenzen der SEQ ID NR: 1 oder 17 (CDR-H3), 3 oder 19 (CDR-H2) und 5 oder 21 (CDR-H1): und
   (ii) die CDRs der V_{L}-Region (CDR-L) die Aminosäuresequenzen der SEQ ID NO: 8 oder 24 (CDR-L3), 10 oder 26 (CDR-L2) und 12 oder 28 (CDR-L1) umfassen oder
      kodiert werden durch die Nukleinsäuresequenzen der SEQ ID NR: 7 oder 23 (CDR-L3), 9 oder 25 (CDR-L2) und 11 oder 27 (CDR-L1); und
(b) eine Nukleinsäuresequenz, die eine konstante Region eines humanem IgG1- oder IgG4-Antikörpers kodiert.

In der Beschreibung werden eine Reihe an Dokumenten inklusive Patentanmeldungen und Hersteller-Gebrauchsanweisungen aus dem Stand der Technik genannt. Während der Offenbarungsgehalt dieser Dokumente als nicht relevant für die Patentfähigkeit der Erfindung angesehen wird, ist er per Referenz in die vorliegende Beschreibung inkorporiert.

Die Stimulation ruhender T-Lymphozyten zur Aktivierung, Proliferation und funktionellen Differenzierung erfordert die Besetzung zweier Oberflächenstrukturen, so genannter Rezeptoren: 1. des Antigenrezeptors, der von Zelle zu Zelle eine unterschiedliche Spezifität besitzt und für die Erkennung von Antigenen, z.B. viralen Spaltprodukten, notwendig ist; sowie 2. des auf allen ruhenden T-Zellen mit Ausnahme einer Untergruppe der CD8 T-Zellen des Menschen gleichermaßen exprimierten CD28 Moleküls, welches natürlicherweise an Liganden auf der Oberfläche anderer Zellen des Immunsystems bindet. Man spricht von der Costimulation der antigenspezifischen Immunreaktion durch CD28. In Zellkultur können diese Vorgänge nachgestellt werden durch Besetzung des Antigenrezeptors sowie des CD28-Moleküls mit geeigneten monoklonalen Antikörpern (mAb). Im klassischen System der Costimulation führt wender die Besetzung des Antigenrezeptors noch die des CD28-Moleküls allein zur T-Zellproliferation, die Besetzung beider Rezeptoren ist jedoch effektiv. Diese Beobachtung wurde an T-Zellen des Menschen, der Maus und der Ratte gemacht.

Allerdings sind auch CD28-spezifische monoklonale Antikörper (mAb) bekannt, die ohne Costimulation die T-Zellproliferation einleiten können. Eine solche superagonistische, d. h. von der Besetzung des Antigenrezeptors unabhängige Aktivierung ruhender T-Lymphozyten durch CD28-spezifische mAk ist beispielsweise aus Tacke et al., Eur. J. Immunol., 1997, 27:239-247, bekannt. Demnach wurden zwei Arten von CD28-spezifischen monoklonalen Antikörpern mit unterschiedlichen funktionellen Eigenschaften beschrieben: costimulatorische mAb, die die Aktivierung ruhender T-Zellen nur bei gleichzeitiger Besetzung des Antigenrezeptors costimulieren, und superagonistische mAb, die ohne Besetzung des Antigenrezeptors T-Lymphozyten aller Klassen in vitro und in Ratten zur Proliferation aktivieren können.

Aus der DE 101 60 516.1 sowie aus Lühder et al., J. Exp. Med., 2003, 197: 955-966, sind darüber hinaus superagonistische mAk mit Spezifität für das CD28 Molekül des Menschen bekannt; welche in vitro ohne Stimulation des T-Zellantigenrezeptors (TCR) T-Zellen sehr effizient aktivieren und expandieren. Hier bediente man sich jedoch immobilisierter Antikörper, die sich, wie weiter unten dargestellt, nicht für therapeutische Anwendung eignen.

Des weiteren zeigten superagonistische anti-CD28 Antikörper Antikörper in Tiermodellen und in Zellkultur ausgeprägte anti-inflammatorische Eigenschaften. So kann beispielsweise, wie in Schmidt et al., J. Neuroimmunol., 2003, 140: 143-152, belegt, die Applikation eines superagonistischen mAk gegen das CD28 Molekül der Ratte die Entstehung einer entzündlichen peripheren Nervenerkrankung, der Experimentellen Autoimmunen Neuritis, EAN, verhindern. Aus der DE 102 12 108.7 sowie aus Lin et al., Eur. J. Immunol., 2003, 33:626-638, ist bekannt, dass superagonistische anti-CD28 Antikörper eine überproportional starke Aktivierung regulatorischer T-Zellen bewirken können, Die Funktion regulatorischer T-Zellen ist, autoaggressive T Zellen zu kontrollieren und dafür Sorge zu tragen, dass es generell zu keiner überschießenden Entzündungsreaktion kommt (Schwartz, Nature Immunol., 2005, 6: 327-330). Allerdings kann ein Eingreifen in die CD28-vermittelte Costimulation das Th1/Th2-Gleichgewicht zugunsten des pro-inflammatorischen Th1-Phänotyps verschieben und birgt daher die Gefahr, Autoimmun-/ entzündliche Reaktionen zu verschlimmern (sh. Schmidt et al., supra).

Für therapeutische Antikörperkandidaten ist es aus nahe liegenden Gründen erstrebenswert, die Immunogenität, d.h. das Auslösen einer Immunantwort gegen die Wirksubstanz, zu vermeiden mit dem Ziel, die pharmakologische Wirksamkeit im Menschen voll auszuschöpfen und gleichzeitig unerwünschte Nebenwirkungen zu reduzieren. Um die Immunogenität von Antikörpern, die nicht aus dem Menschen stammen, zu vermeiden, ist beispielsweise die "Humanisierung" von Antikörpern mittels gentechnologischer Methoden Stand der Technik. Hierbei wird die Antigenbindungsstelle eines ursprünglich nicht aus dem Menschen stammenden Antikörpers konserviert, während der Rest des Antikörpermoleküls, insbesondere die konstanten Anteile der Antikörper (konstante Domäne oder Fc-Fragment) gegen eine strukturähnliche Variante aus dem menschlichen Erbgut ausgetauscht wird. (Hwang et al., Methods, 2005, 36: 3-10).

Wie aus der DE 102 30 223.5 bekannt ist, verstärkt die Quervernetzung (oder Kreuzvernetzung, "cross-linking") superagonistischer anti-human CD28 Antikörper deren Fähigkeit, T-Lymphozyten in einer Zellsuspension zu aktivieren. Beispielsweise wird die Proliferation gereinigter T-Lymphozyten um ein vielfaches verstärkt, wenn superagonistische Antikörper in Form von immobilisierten Molekülkomplexen auf paramagnetischen Kügelchen verwendet werden, statt in der T-Zellsuspension in löslicher Form vorzuliegen. Für die galenische Anwendung im Menschen ist die Applikation solchermaßen komplexierter Darreichungsformen von superagonistischen anti-CD28 Antikörpern aus nahe liegenden Gründen jedoch nicht möglich. Des weiteren kamen in der DE 102 30 223.5 auf paramagnetischen Kügelchen immobilisierte anti-Maus-IgG-Antikörper als kreuzvernetzendes Agens zum Einsatz. Auch in dieser Hinsicht kommt der analoge Ansatz für eine therapeutische Anwendung im Menschen nicht in Frage, da sich die Verwendung von anti-human-IgG-Antikörpern auf Grund der Fülle der zu erwartenden Kreuzreaktionen verbietet. Nicht zuletzt ist der in der DE 102 30 223.5 beschriebene Ansatz auf ein in vitro Verfahren, bei dem gereinigte T-Zellen zum Einsatz kommen, beschränkt. Für die Entwicklung therapeutischer superagonistischer anti-CD28 Antikörper war demzufolge zu prüfen, ob ein geeignetes Antikörperformat existiert, das in vivo eine hinreichende Kreuzvemetzung ermöglicht, ohne unerwünschte Effekte zu erzielen.

Es ist bekannt, dass eine natürliche Erkennung und Quer- oder Kreuzvemetzung von Fc Domänen von Antikörpern im menschlichen Körper durch Fc-Rezeptoren, die auf unterschiedlichen Zelltypen ausgeprägt werden, vermittelt wird (Woof et al., Nature Reviews Immunol., 2004, 1-11). Ziel der Fc-Rezeptor-vermittelten Antikörper-Bindung im physiologischen Kontext ist das "aus-dem-Verkehr-Ziehen" oder Zerstören von antikörperbeladenen Zellen, da davon auszugehen ist, dass Antikörper nur gegen solche Zellen bebildet werden, die von fremdem Gewebe stammen, bakteriell oder viral infiziert, gestresst oder bösartig entartet sind. Wichtige Fc-Rezeptor-vermittelte Mechanismen der Eliminierung antikörperbeladener Zellen sind die komplementabhängige Zytotoxizität (CDC), die antikörperabhängige zelluläre Zytotoxizität (ADCC) und die Opsonisierung, d.h. Kenntlichmachung zur Phagozytose durch spezialisierte Fresszellen.

Die Fc-Rezeptoren, die für die Erkennung von Fc-Domänen menschlicher Antikörper des IgG Isotyps verantwortlich sind, lassen sich in die Gruppen CD64 (Fc gamma Rezeptor I; hoch-affiner Rezeptor); CD32 (Fc gamma Rezeptor II; intermediär-affiner Rezeptor) und CD16 (Fc gamma Rezeptor III; niedrig-affiner Rezeptor) klassifizieren. Ihre Bindungseigenschaften an Antikörper der IgG Untergruppen IgG1, IgG2, IgG3 und IgG4 sowie die durch die Bindung an diese Antikörper ausgelösten Effektorfunktionen sind weitgehend bekannt (Woof et al., Nature Reviews Immunol., 2004, 1-11). So bewirkt die Stimulation von Fc-Rezeptoren, die an IgG1 bzw. IgG4 binden (CD64 stark an IgG1, mäßig an IgG4; CD32 schwach an IgG1, nicht an IgG4; CD16b stark an IgG1, sehr schwach oder nicht an IgG4), in der Regel auch eine Eliminierung der Zielzellen über ADCC oder CDC.

Zusammenfassend ist somit aus dem Stand der Technik bekannt, dass Quervernetzung, wenn sie durch im menschlichen Körper vorhandene Moleküle bewirkt wird, überwiegend mit einer Eliminierung der antikörperbeladenen Zellen einhergeht.

Demzufolge bestand die Aufgabe bei der Entwicklung therapeutischer superagonistischer anti-CD28 Antikörper darin, ein Antikörperformat zu finden, das sich zum einen im Körper durch physiologisch vorhandene Strukturen oder Moleküle, beispielsweise Fc-Rezeptoren, kreuzvemetzten lässt. Zum anderen soll eine solche Kreuzvemetzung aber nicht zu einer (wesentlichen) Eliminierung oder Zerstörung der eigentlichen Zielstruktur des Antikörpers, nämlich den CD28⁺ T-Zellen führen. Diese Aufgabe wird durch die in den Ansprüchen bereitgestellten Ausführungsformen gelöst.

Dementsprechend betrifft die Erfindung eine Nukleinsäure oder mehrere Nukleinsäuren; kodierend ein Bindungsmolekül, das spezifisch ein menschliches CD28-Molekül bindet, umfassend
(a) eine Nukleinsäuresequenz kodierend eine V_{H}-Region und eine Nukleinsäuresequenz kodierend eine V_{L}-Region umfassend CDRs in einem human Immunglobulin-Gerüst, wobei
   (i) die CDRs der V_{H}-Region (CDR-H) die Aminosäuresequenzen der SEQ ID NO: 2 oder 18 (CDR-H3), 4 oder 20 (CDR-H2) und 6 oder 22 (CDR-H1) umfassen oder kodiert werden durch die Nukleinsäuresequenzen der SEQ ID NR: 1 oder 17 (CDR-H3), 3 oder 19 (CDR-H2) und 5 oder 21 (CDR-H1); und
   (ii) die CDRs der V_{L}-Region (CDR-L) die Aminosäuresequenzen der SEQ ID NO: 8 oder 24 (CDR-L3), 10 oder 26 (CDR-L2) und 12 oder 28 (CDR-L1) umfassen
      oder
      kodiert werden durch die Nukleinsäuresequenzen der SEQ ID NR: 7 oder 23 (CDR-L3), 9 oder 25 (CDR-L2) und 11 oder 27 (CDR-L1); und
(b) eine Nukleinsäuresequenz, die eine konstante Region eines humanem IgG1- oder IgG4-Antikörpers kodiert.

Der Begriff "Nukleinsäure" im Zusammenhang mit der vorliegenden Erfindung bezieht sich auf ein Nukleinsäuremolekül oder mehrere Nukleinsäuremoleküle, die in ihrer Gesamtheit das erfindungsgemäße Bindungsmolekül kodieren. Die kodierenden Regionen für die Komponenten des erfindungsgemäßen Bindungsmolekül können somit von einem Nukleinsäuremolekül umfasst sein oder sich auf distinkten, zwei oder mehr als zwei Nukleinsäuremolekülen befinden. Insbesondere können sich die kodierenden Bereiche für die V_{H}-Region und die konstante Region der schweren Kette des humanen IgG1 oder IgG4 auf einem Nukleinsäuremolekül befinden und die V_{L}-Region und die konstante Region der leichten Kette des IgG1 oder IgG4 auf einem zweiten Nukleinsäuremoleküle befinden. Die konstante Region der leichten Kette kann sowohl der Sequenz eines κ-Gens als auch eines λ-Gens entsprechen, die für die konstanten Regionen menschlicher leichter Ketten kodieren. Entsprechend der Erfindung ist im Bindungsmolekül, das von der/den erfindungsgemäßen Nukleinsäure(n) kodiert wird, die V_{H}-Region bzw. V_{L}-Region mit der konstanten Region funktionell verbunden, wie dies beispielsweise in einem Antikörper der Fall ist. Ebenso versteht es sich, dass sich die kodierte(n) (Poly)peptidkette(n) nach ihrer Synthese so falten bzw. assemblieren, dass die V_{H}-Region und die V_{L}-Region in räumliche Nachbarschaft gelangen und eine Antigen-Bindungsstelle ausbilden. Auch dies ist durch einen Antikörper exemplifiziert.

Der Begriff "umfasst" bedeutet einerseits "enthält" (neben anderen Gegenständen) und andererseits "besteht daraus" (ohne den Einschluss weiterer Gegenstände).

Das CD28-Molekül ist, wie oben beschrieben, ein transmembranes Molekül, das als Homodimer auf der Oberfläche von T-Zellen exprimiert wird. Die Aminosäuresequenz von humanem CD28 kann unter der GenBank Accession No. NM_006139 abgerufen werden.

Der Ausdruck "superagonistisch" beschreibt im Zusammenhang mit der vorliegenden Erfindung eine Eigenschaft erfindungsgemäßer Bindungsmoleküle, die durch spezifische Bindung an/Interaktion mit einem bestimmten Epitop des CD28-Moleküls eine Antigenrezeptor-unabhängige Aktivierung von Lymphozyten ermöglichen. Somit entspricht eine "superagonistische Stimulation" der Aktivierung von CD28⁺ T-Zellen, ohne dass eine Costimulation, d.h. ein weiteres Bindungsereignis neben einer Bindung/Interaktion des CD28-spezifischen Antikörpers zur Stimulation der Proliferation erforderlich ist. Eine solche Aktivierung kann u.a. über eine Detektion von Aktivierungsmarkern auf den Zellen, die Induktion von Transkriptionsfaktoren, einer Proliferation oder der Expression oder Sezemierung von Zytokinen nachgewiesen werden.

Mit Aktivierung und/oder Expansion von T-Zellen ist insbesondere die Vermehrung der Stoffwechselaktivität, Vergrößerung des Zellvolumens, Synthese immunologisch wichtiger Moleküle, wie CD71 oder CD25, und Eintritt in die Zellteilung (Proliferation) auf einen äußeren Reiz hin bezeichnet. Bevorzugt liegen im Ergebnis nach der Aktivierung bzw. Expansion mehr T-Zellen vor als vorher.
Die Begriffe "V_{H}-Region" und "V_{L}-Region" sind dem Fachmann allgemein bekannt und beschreiben im Stand der Technik die aminoterminale Domäne eines Antikörpers, die während der B-Lymphozytenreifung durch Rekombination der V-, D- und J-Gensegmente entsteht. Die variablen Regionen der schweren und der leichten Kette von Antikörpern sind für die spezifische Bindung/Interaktion eines Antikörpers an/mit dessen spezifischen Epitop verantwortlich (vgl. Haseman und Capra "immunöglobulins: Structure and Function", in "Fundamental Immunology" (ed. W. E. Paul), Raven Press, New York, 2. Ausgabe 1989). Die V_{H}- und V_{L}-Regionen können über rekombinante Verfahren auch mit anderen Strukturen als den natürlicherweise auftretenden C-Regionen kombiniert werden.

Der Begriff "CDR" (complementarity determining regions) beschreibt komplementaritätsbestimmende Regionen der Rezeptoren des Immunsystems, insbesondere von Antikörpern und T-Zellrezeptoren. Diese sind dem Fachmann als Bereiche eines Rezeptors bekannt, die mit dem Liganden in Kontakt treten und die Spezifität des Rezeptors bestimmen. Die CDRs sind die variabelsten Teile der Rezeptoren und für deren Vielfalt verantwortlich. Beispiele für solche Rezeptoren umfassen Antikörper. Je drei der Schleifen befinden sich an den distalen Enden der beiden variablen Domänen von Antikörpern. Diese werden allgemein als CDR-H1 bis CDR-H3 auf der schweren Kette und CDR-L1 bis CDR-L3 auf der leichten Kette von Antikörpern durchnumeriert.

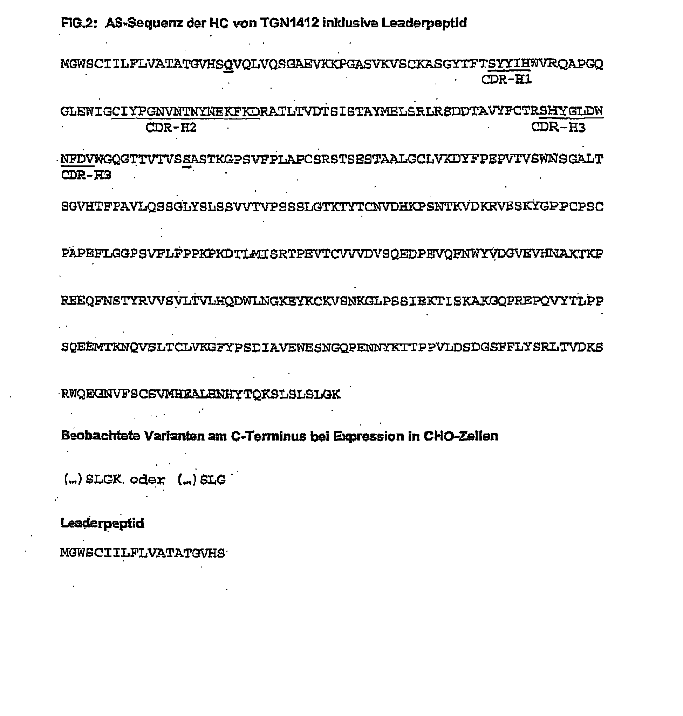

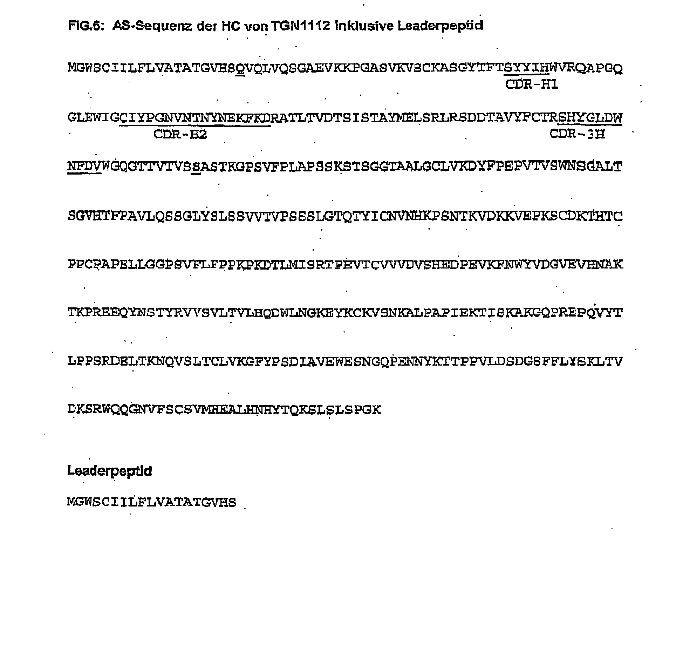

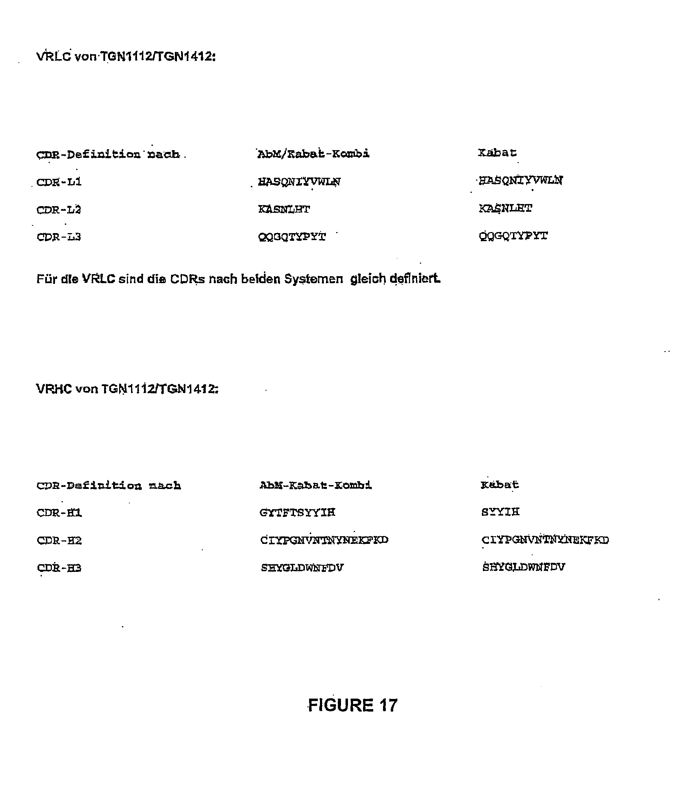

Die Aminosäuresequenz einer CDR oder mehrerer CDRs kann der Fachmann aus einer bekannten Aminosäuresequenz eines Antikörpers, Antikörperfragments oder Derivats bestimmen. Die oben genannten CDRs sind entsprechend dem modifizierten Kabat-Verfahren (AbM/Kabat-Kombi) ermittelt worden. Dieses ergibt sich u.a. aus den Regeln "How to identify the CDRs by looking at a sequence" auf der Homepage von Andrew C.R. Martin (http://www.bioinf.org.uk/abs/). Alternativ können erfindungsgemäß Regeln zur Ermittlung der CDRs der beschriebenen Bindungsmoleküle gemäß Kabat (sh. Kabat, E. A. et al. 1991; Sequences of Proteins of Immunological Interest. Fifth Edition. NIH Publication No. 91-3242, sowie Johnson, G. and Wu, T. T. 2000, Nucleic Acids Research) verwendet werden. In diesem Falle gelten in den erfindungsgemäßen Ausführungsformen die nach Kabat ermittelten und in Figur 17 dargestellten sowie in den Figuren 2 und 6 hervorgehobenen CDR-Sequenzen. Das bedeutet ferner, dass sich die Framework-Regionen leicht verändern, ohne dass sich jedoch die Gesamtsequenz des Bindungsmoleküls ändert oder ändern müsste. Wie erwähnt: Die CDRs der erfindungsgemäßen Bindungsmoleküle gemäß des Kabat-Systems, als auch des AbM/Kabat-Kombi-Systems sind in der Figur 17 dargestellt. Beispielsweise ist die CDR-H1 Sequenz nach dem modifizierten Kabat-System durch die Sequenz GYTFTSYYIH gegeben, nach dem herkömmlichen Kabat-Verfahren durch SYYIH.

Der Begriff "humanes Immunglobulin-Gerüst ("Immunglobulin-Framework") beschreibt die Gesamtheit jener Aminosäuresequenzabschnitte im gefalteten Zustand, die in der variablen Region eines humanen Antikörpers zwischen den CDRs sowie N- und C-terminal hiervon liegen und das räumliche Gerüst (sterische Formation) des Immunglobulins vorgeben.

Der Begriff konstante Region ist dem Fachmann u.a. aus Janeway und Travers (Immunologie, 2. Auflage, Spektrum Akademischer Verlag, s. a. Referenz W. E. Paul, supra) bekannt. Humane Antikörper des Isotyps IgG umfassen die Subklassen IgG1, IgG2, IgG3 und IgG4. Erfindungsgemäß kommt in den Bindungsmolekülen eine konstante Region eines humanem IgG1- oder IgG4-Antikörpers zum Einsatz. Der Begriff "binden an/interagieren mit" wird im Zusammenhang mit der vorliegenden Erfindung definiert als Bindung/Interaktion von einer "Antigen-Bindungsstelle" mit einem Epitop. Der Begriff "Antigen-Bindungsstelle" definiert, in Übereinstimmung mit der vorliegenden Erfindung, ein Motiv eines Polypeptids/Bindungsmoleküls, das geeignet ist, mit einem spezifischen Antigen oder einer Gruppe von Antigenen zu interagieren. Eine solche spezifische Bindung/Interaktion wird auch als ein "spezifisches Erkennen" eines Antigens definiert. Eine spezifische Erkennung eines Antigens ist erfindungsgemäß eine spezifische Bindung an/Interaktion mit mindestens zwei, vorzugsweise drei, weiter bevorzugt mit mindestens vier Aminosäuren eines Antigens. Der Teil des Antigens, der die Bindung/Interaktion mit der Antigen-Bindungsstelle eingeht, wird als Epitop bezeichnet. Das Antigen ist für die erfindungsgemäßen Bindungsmoleküle das CD28-Molekül. Das Epitop der erfindungsgemäßen Bindungsmoleküle befindet sich im als C'D-loop bezeichneten Bereich des CD28-Moleküls. Diese spezifische Bindung/Interaktion führt zur Induktion eines superagonistischen Signals (Aktivierung/Stimulation) in einer CD28⁺ Zelle. Eine spezifische Bindung/Interaktion kann auch mit dem "Schlüssel-Schloss-Prinzip" beschrieben werden. Spezifische Motive in einer Aminosäuresequenz der Antigen-Erkennungsstelle und des Antigens binden aneinander aufgrund ihrer primären, sekundären oder tertiären Struktur. Die Begriff "spezifische Interaktion", wird im Zusammenhang mit der vorliegenden Erfindung so verstanden, dass das Bindungsmolekül nicht oder nicht signifikant kreuz-reagiert mit (Poly)peptiden, die eine ähnliche Struktur wie das spezifisch erkannte Antigen haben und auf den selben Zellen exprimiert werden. Die Kreuzreaktivität einer Gruppe von Bindungsmolekülen kann z.B. durch Bestimmung der Bindungseigenschaften/Bindungsstärken unter üblichen Bedingungen untersucht werden (siehe u.a. Harlow and Lane, Antibodies: A Laboratory Manual, Cold Spring Harbor Laboratory Press, 1988 und Using Antibodies: A Laboratory Manual, Cold Spring Harbor Laboratory Press, 1999). Explizit umfasst sind durch die Definition des "Bindens an/Interagieren mit" auch Konformationsepitope, strukturelle Epitope und diskontinuierliche Epitope, die aus zwei oder mehr Bereichen eines Antigens zusammengesetzt sind (Reste unterschiedlicher Bereiche einer oder mehrer Polypeptidketten) und ein "natürliches Epitop" zusammensetzen (siehe Sela, (1969) Science 166, 1365 und Laver, (1990) Cell 61, 553-6).

Der Begriff "Kreuzvernetzung/Quervernetzung" beschreibt eine Herbeiführung einer räumlichen Nähe mehrerer Bindungsmoleküle, die an das spezifische Antigen gebunden sind/mit dem spezifischen Antigen interagieren, wodurch es zu einer multivalenten Bindung des Zielmoleküls (spezifischen Epitops) CD28 kommt. Bei den erfindungsgemäßen Bindungsmolekülen ist eine Kreuzvernetzung/Quervernetzung über das F_{C}-Fragment des humanisierten IgG-Antikörpers bevorzugt. Dies kann z.B. durch Bindung dieses Fragments an F_{C}-Rezeptoren auf Lymphozyten oder anderen Zellen erreicht werden. Erfindungsgemäße Bindungsmoleküle (die durch erfindungsgemäße Nukleinsäuren kodiert werden) haben die überraschende Eigenschaft, dass CD28⁺ Zellen durch kreuzvemetzte Bindungsmoleküle aktiviert/stimuliert werden können, ohne eliminiert zu werden.

Der Begriff "Bindungsmolekül" wird im Zusammenhang mit der Erfindung zur Beschreibung einer Gruppe von Molekülen verwendet, die sowohl als Monomere, als auch als Di-, Tri- oder Oligomere auftreten können. Di-, Tri- oder Oligomere können sowohl Homomere, als auch Heteromere sein. Vorzugsweise sind die einzelnen Ketten von Di-, Tri- oder Oligomere kovalent miteinander verbunden. Insbesondere bevorzugt sind hierbei kovalente Verbindungen über Disulfidbrücken. Alternativ können die Di-, Tri- oder Oligomere aber auch über nicht-kovalente Wechselwirkungen miteinander verbunden sein. Entsprechende Di- Tri- oder Oligomerisierungen können z.B. durch Wechselwirkungen spezifischer Sequenzmotive in den einzelnen Molekülen zustande kommen. Ein Beispiel für eine solche intrinsische Wechselwirkung ist die Wechselwirkung von Monomeren durch ein "Leucin-Zipper-Motiv", welches zu einer Dimerisierung führt.

Als Ausgangspunkt für die erfindungsgemäßen superagonistischen CD28-Bindungsmoleküle diente der in Luhder et al., J. Exp. Med., 2003, 197: 955-966, beschriebene Maus anti-human CD28 Antikörper 5.11A1. Die variablen Regionen der schweren und leichten Kette des 5.11A1 Antikörpers wurden dem Stand der Technik entsprechend gentechnolgisch humanisiert. Diese Technik ist u.a. als "CDR-grafting" dem Fachmann bekannt (sh. P.T. Jones et al. 1986. Nature 321: 522-525 sowie Ewert et al., 2004. Methods, 34: 184-199). Die humanisierten variablen Regionen wurden mit der konstanten Region humaner Antikörper des IgG-Isotyps fusioniert.

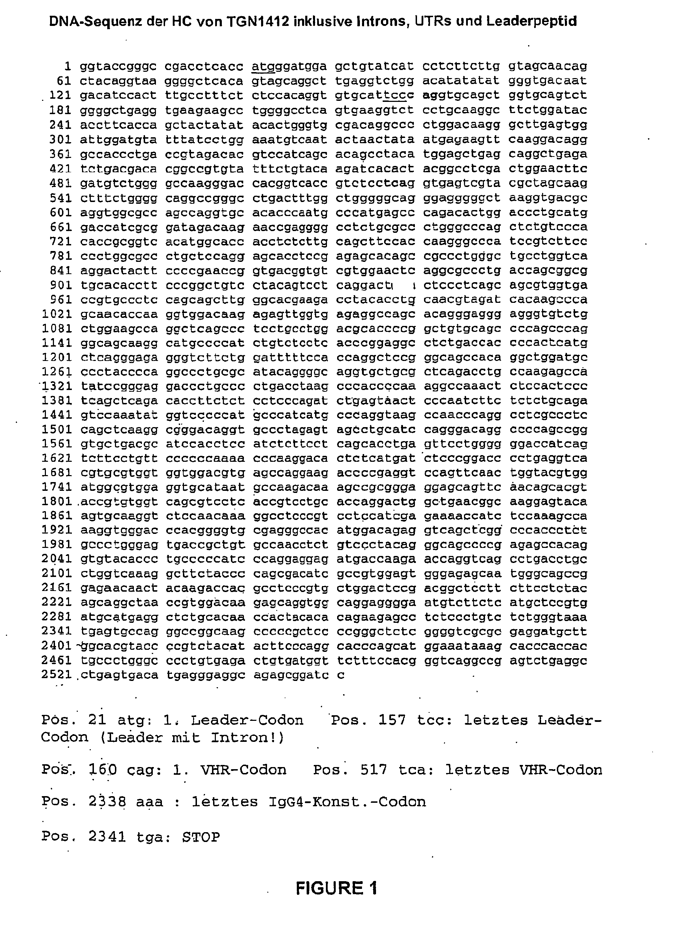

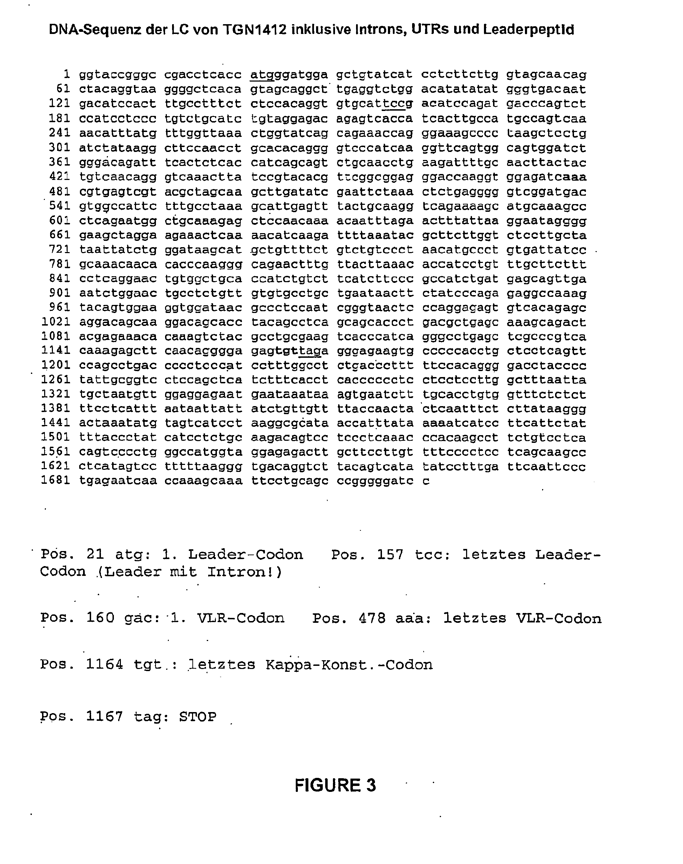

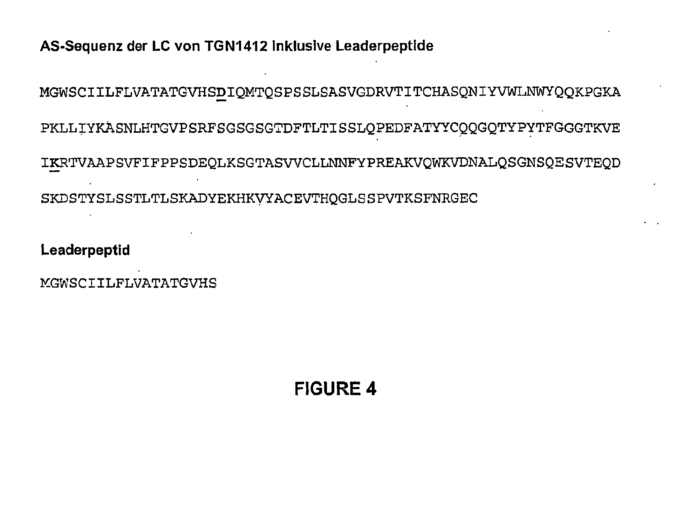

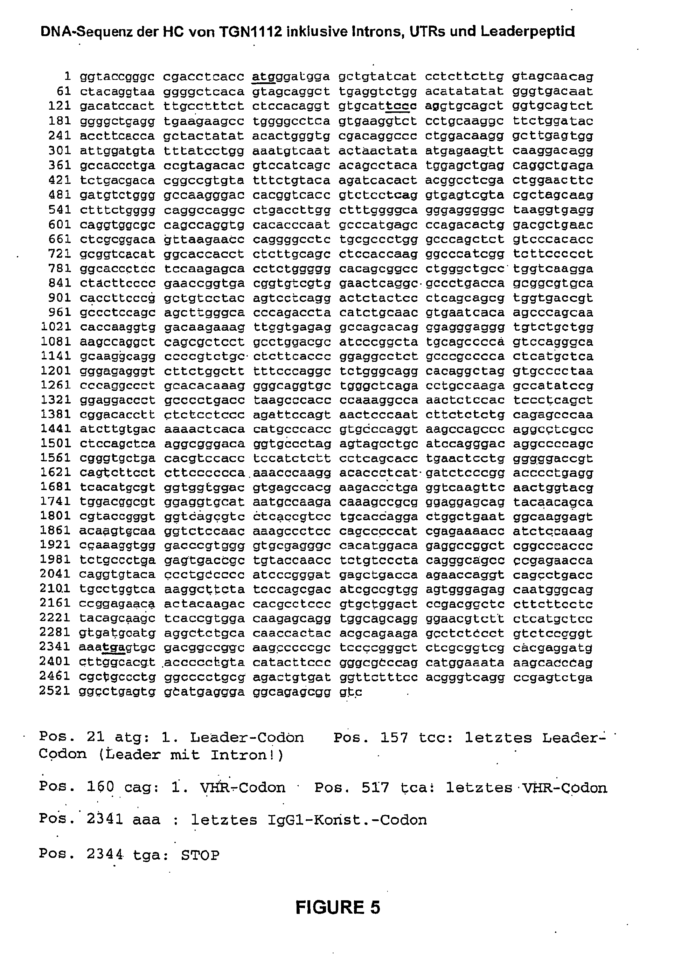

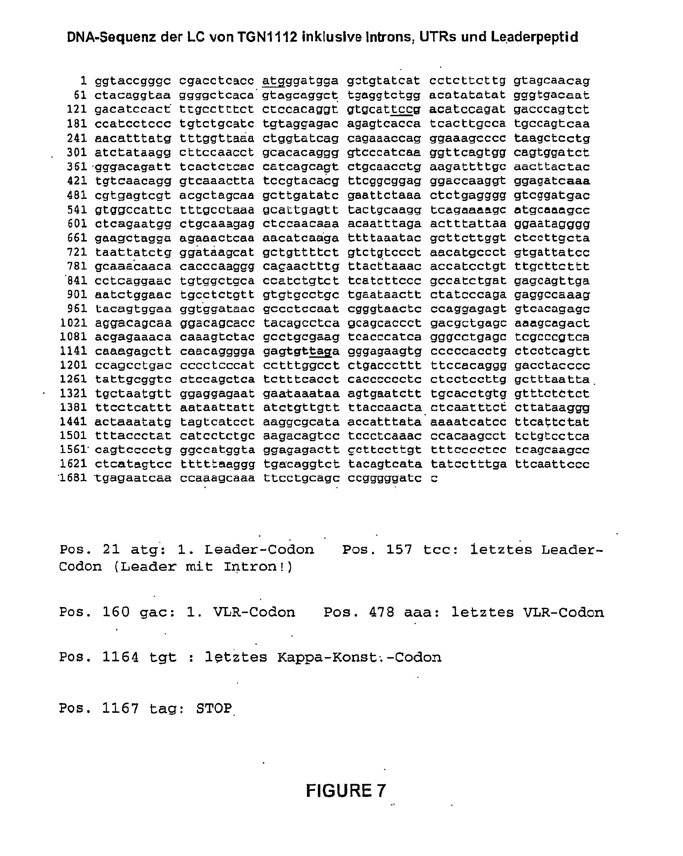

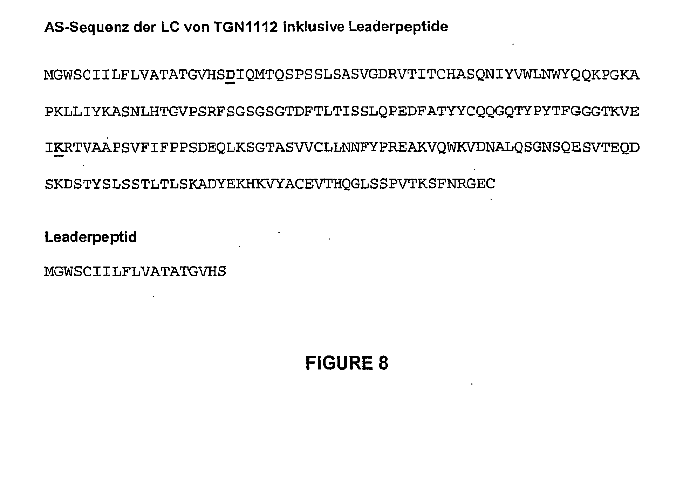

Es ist bekannt, dass für den therapeutischen Einsatz in Prinzip alle IgG Isotypen (IgG1-4) geeignet sind. Bekanntermaßen besitzen die große Mehrheit der sich in der therapeutischen Entwicklung befindlichen Antikörper ein neutralisierendes, blockierendes oder zerstörerisches (negatives) Wirkprinzip und unterscheiden sich dadurch grundlegend von dem hier verfolgten superagonistischen (positiven) Wirkprinzip. Die erfindungsgemäße Auswahl der Isotypen IgG1 und IgG4 als therapeutische Formate von superagonistischen anti-CD28 Antikörpern beruht auf folgenden Überlegungen und überraschenden experimentellen Befunden: Basis des erfinderischen Prinzips ist die Auswahl von F_{C}-Fragmenten des IgG1 bzw. IgG4 Isotyps. IgG1-Antikörper können mittels im Körper vorhandener F_{C}-Rezeptoren effizient quervernetzt werden. Dies sollte dem superagonistischen Wirkprinzip zuträglich sein (siehe auch Evans et al., Nature Immunol., 2005, 6: 271-279). Dagegen sprach allerdings, dass zu erwarten war, dass eine solche Quervernetzung zu einer Eliminierung der T-Zellen führen würde, was dem gewünschten Wirkprinzip entgegen stünde bzw. zu erwarten stand, dass das gewünschte Wirkprinzip durch diese Eliminierung zunichte gemacht würde. Im Falle des IgG4 Isotyps war dem Stand der Technik zufolge zu erwarten, dass dieser Isotyp der gewünschten Wirksamkeit gegenläufig ist. Für diesen war z.B. eine antikörperabhängige zelluläre Zytotoxizität (ADCC) auslösende Wirkung beschrieben; siehe Isaacs et al., Clin. Exp. Immunol., 106: 427-433. Darüber hinaus war aufgrund der zu erwartenden fehlenden oder sehr geringen Fc-Rezeptor Bindung nicht davon auszugehen, dass dieser Isotyp eine T-Zell-Aktivierung beispielsweise in einer Kultur von peripheren Blutleukozyten fördern würde.
Es wurde nach Bereitstellung der erfindungsgemäßen Bindungsmoleküle überraschenderweise festgestellt, dass diese superagonistisch T-Zellen stimulieren. Dies ist für die Antikörper des IgG1 Isotyps (TGN1112) und des IgG4 Isotyps (TGN1412) in den beigefügten Beispielen und den in den Figuren 1 bis 8 dargestellten experimentellen Ergebnissen gezeigt. Die schwere und leichte Kette von TGN1412 ist jeweils in SEQ ID NR: 14 und 16 dargestellt. SEQ ID NR: 30 und 32 zeigen die Aminosäuresequenzen der schweren und leichten Kette von TGN1112. Wie den Figuren 1 bis 8 zu entnehmen ist, werden sowohl die leichten als auch die schweren Ketten der Antikörper TGN1112 und TGN1412 zunächst mit Signalpeptid (in den Figuren als Leaderpeptid bezeichnet) synthetisiert. Das Signalpeptid bestimmt das Targeting innerhalb der Zelle und ist auf der reifen leichten bzw. schweren Kette nicht mehr vorhanden. Hinsichtlich der schweren Kette von TGN1412 ist noch anzumerken, dass bei der Expression in CHO-Zellen zwei Varianten des C-Terminus beobachtet wurden, die sich um einen zusätzlichen Aminosäurerest unterscheiden (Fig. 2). Die DNA-Sequenzen einschließlich Introns und UTR und für das Signalpeptid kodierender Region sind für die schwere und leichte Kette von TGN1412 in SEQ ID NR: 41 und 43 dargestellt; für schwere und leichte Kette von TGN1112 in SEQ ID NR: 45 und 47. Die davon kodierten Aminosäuresequenzen sind in SEQ ID NR: 42, 44, 46 und 48 dargestellt.

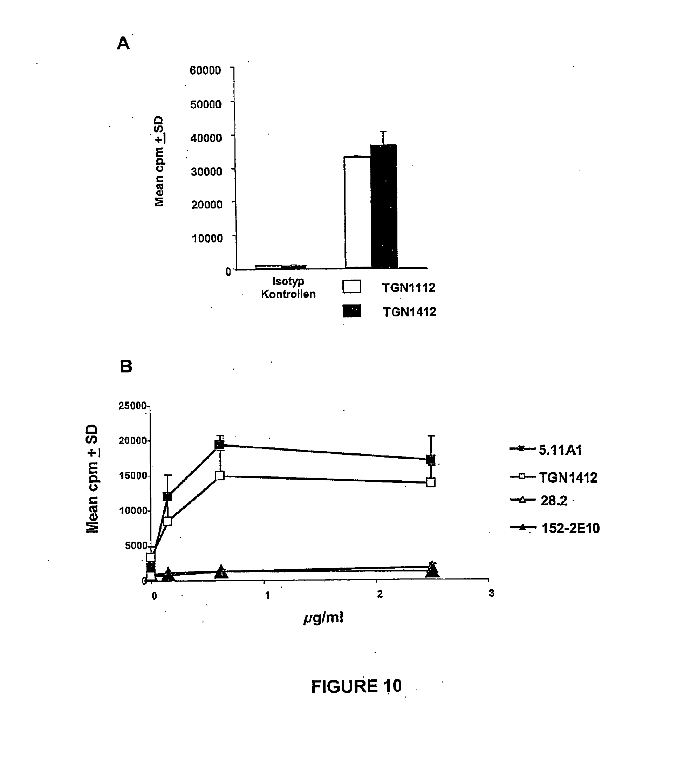

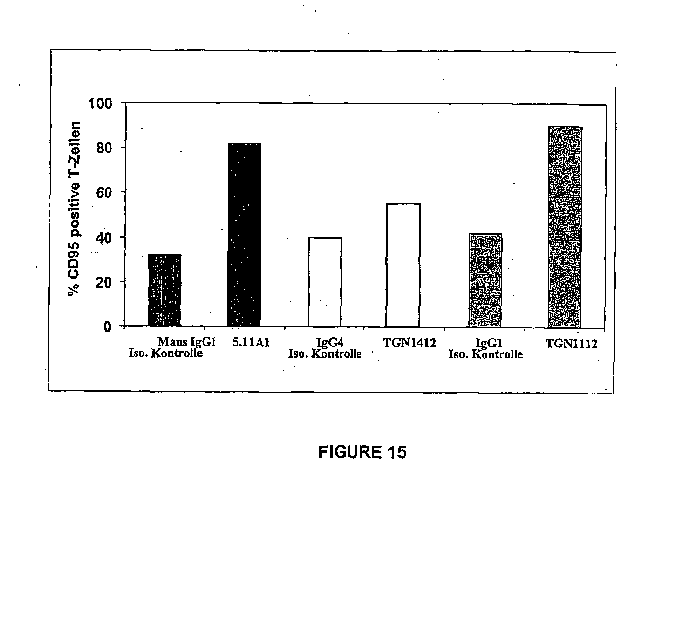

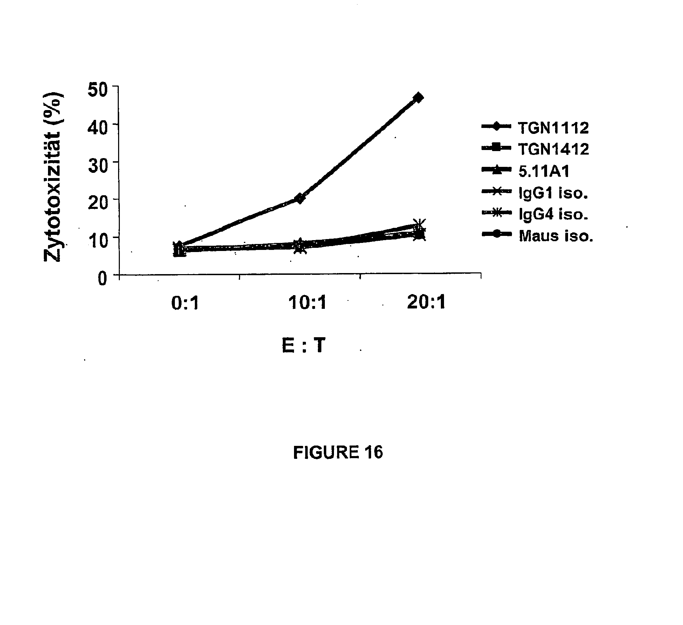

Die vorliegenden Erfindung stellt somit in einer bevorzugten Ausführungsform die löslichen anti-CD28 Antikörper TGN1412 und TGN1112 zur polyklonalen Stimulation menschlicher CD4 und CD8 T-Lymphozyten auf eine neuartige, TCR unabhängig Art und Weise zur Verfügung. Unerwartet war der Befund, dass TGN1412 und TGN1112 in löslicher Form die T-Lymphozyten ex vivo sehr effizient aktivieren und expandieren. Dieser Befund war insbesondere für TGN1412 überraschend, da einerseits Quervernetzung des Antikörpers für das superagonistische Wirkprinzip notwendig ist, andererseits aber keine Bindung an hoch- oder intermediär-affine F_{C}-Rezeptoren nachweisbar war. Für TGN1112 war der Befund überraschend, da der Antikörper in Zellkultur eine stark ausgeprägte Antikörper-abhängige Zytotoxizität gegenüber T-Zellen vermittelt (Fig.16), jedoch gleichzeitig eine sehr starke Proliferation induziert (Fig. 10A). Gegenüber dem Ausgangsantikörper 5.11A1 zeichnen sich sowohl TGN1412 als auch TGN1112 dadurch aus, dass sie in der Lage sind, den aktivierungsinduzierten Zelltod (Apoptose) menschlicher T-Lymphozyten in Zellkultur sehr niedrig zu halten (Fig. 15). Dieses Ergebnis stellt in Aussicht, dass auch im Menschen TGN1412 und TGN1112 in der Lage sein sollten, T-Lymphozyten sehr effektiv und schonend zu aktivieren und zu expandieren.

Die erfindungsgemäße Nukleinsäure oder die erfindungsgemäßen mehreren Nukleinsäuren umfasst/umfassen vorzugsweise eine Nukleinsäuresequenz kodierend eine V_{H}-Region und eine Nukleinsäuresequenz kodierend eine V_{L}-Region, wobei
(i) die Nukleinsäuresequenz der V_{H}-Region SEQ ID NR: 33 oder 37 umfasst und/oder ein (Poly)peptid kodiert, das die Aminosäuresequenz SEQ ID NR: 34 oder 38 umfasst; und
(ii) die Nukleinsäuresequenz der V_{L}-Region SEQ ID NR: 35 oder 39 umfasst und/oder ein (Poly)peptid kodiert, das die Aminosäuresequenz SEQ ID NR: 36 oder 40 umfasst.

Die erfindungsgemäße Nukleinsäure oder die erfindungsgemäßen mehreren Nukleinsäuren umfasst/umfassen weiter bevorzugt eine Nukleinsäuresequenz, wobei die Nukleinsäuresequenz
(i) SEQ ID NR: 13, 29, 41 oder 45 ist; und/oder
(ii) ein (Poly)peptid mit der Aminosäuresequenz SEQ ID NR: 14, 30, 42 oder 46 kodiert.
Ebenso bevorzugt sind eine Nukleinsäure oder mehrere Nukleinsäuren, die eine Nukleinsäuresequenz umfassen, wobei die Nukleinsäuresequenz
(i) SEQ ID NR: 15, 31, 43 oder 47 ist; und/oder
(ii) ein (Poly)peptid mit der Aminosäuresequenz SEQ ID NR: 16, 32, 44 oder 48 kodiert.

Es ist darüber hinaus ein bevorzugter Gegenstand der Erfindung, dass eine erfindungsgemäße Nukleinsäure oder die mehreren erfindungsgemäßen Nukleinsäuren zusätzlich eine Nukleinsäuresequenz umfasst/umfassen, die für ein Markierungselement oder Tag kodiert.
Der Begriff "Markierungselement" oder "Tag" beschreibt im Zusammenhang mit der Erfindung ein Element, das die Fähigkeit zur Wechselwirkung mit einem bekannten Bindungspartner vermittelt. Diese Wechselwirkung ihrerseits eröffnet Anwendungen wie (erleichterte) Aufreinigung oder Isolierung sowie Detektion oder Nachweis.
Die erfindungsgemäßen Markierungselemente oder Tags können beispielhaft ausgewählt sein aus einer Gruppe bestehend aus His-tag, Flag-tag, Myc-tag, HA-tag, GST-tag, T100™, VSV-G, V5, S-tag™, HSV, CFP, RFP, YFP, GFP, BFP, Cellulose binding domain (CBD), Maltose binding protein (MBP), NusA-tag, thioredoxin (Trx), DsbA, DabC und einer Biotinylierungssequenz. Alternativ dazu bzw. insbesondere kann das Markierungselement eine radioaktive, fluoreszierende, phosphoreszierende oder lumineszierende Markierung sein. Radioaktive Markierungen schließen Markierungen mit ³²P (bei Nukleinsäuren) und ¹²⁵I oder ¹³²I (bei Proteinen) ein.

In einer alternativen Ausführungsform betrifft die vorliegende Erfindung einen Vektor umfassend eine oder mehrere der oben beschriebene erfindungsgemäßen Nukleinsäuren.

Dem Molekularbiologen als Fachmann sind eine Vielzahl von geeigneten Vektoren bekannt. Die Wahl eines Vektors hängt in diesem Zusammenhang von den gewünschten Eigenschaften ab und schließt Plasmide, Cosmide, Viren, Bakteriophagen und andere konventionell in der Gentechnologie verwendete Vektoren ein. Allgemein bekannte Verfahren können zur Herstellung verschiedener Vektoren verwendet werden; siehe u.a. Sambrook et al. Molecular Cloning: A Laboratory Manual; Cold Spring Harbor Laboratory Press, 2nd edition 1989 and 3rd edition 2001; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002 und Ausubel, Current Protocols in Molecular Biology, Green Publishing Associates and Wiley Interscience, N.Y. (1989), (1994).
Zu exprimierende Nukleotidsequenzen können in geeignete Expressionsvektoren kloniert werden. Übliche Klonierungsvektoren umfassen pBluescript SK, pGEM, pUC9, pBR322 und pGBT9. Übliche Expressionsvektoren umfassen u.a. pTRE, pCAL-n-EK, pESP-1, pOP13CAT, pKNOH2 und pLNOK (Norderhaug et al., 1997. J. Immunol. Methods, 204: 77-87).

Ein erfindungsgemäßer Vektor umfasst üblicherweise eine "regulatorische Sequenz", die funktionell mit der zu exprimierende Nukleotidsequenzen verküpft ist. Der Begriff "regulatorische Sequenz" beschreibt DNA Sequenzen, die notwendig sind, um die Expression einer kodierten Sequenz zu initiieren. Die Eigenschaften solcher Kontrollsequenzen unterscheiden sich in Abhängigkeit von einem Wirtsorganismus. Der Begriff "funktionell verknüpft" beschreibt eine Anordnung, in der die genannten Komponenten in für die Expression geeigneter Weise angeordnet sind. Die geeignete Anordnung ist dem Fachmann bekannt.

Vorzugsweise ist der beschriebene Vektor ein Expressionsvektor. Ein Expressionsvektor ist ein Konstrukt, das zur Transformation eines ausgewählten Wirtes geeignet ist und eine Expression der kodierten Sequenz in dem Wirt ermöglicht. Expressionsvektoren können entsprechend Klonierungsvektoren, binäre Vektoren oder integrierte Vektoren sein. Expression umfasst die Transkription der Nukleinsäure, vorzugsweise in translatierbare mRNA. Regulatorische Elemente, die eine Expression in Prokaryoten und/oder eukaryotischen Zellen gewährleisten sind dem Fachmann bekannt. Kontrolisequenzen für Prokaryoten umfassen im allgemeinen einen Promotor, eine ribosomale Bindungsstelle und einen Terminator. Mögliche regulatorische Elemente für prokaryotische Wirte umfassen z.B. PL, lac, trp or tac Promotoren aus E. coli. Kontrollsequenzen für eukaryotische Zellen umfassen im allgemeinen Promotoren und Terminatoren und unter Umständen Enhancer, Transaktivatoren oder Transkriptionsfaktor-Bindungsstellen. Promotoren gewährleisten eine Initiation der Transkription. Terminatoren, wie poly-A Signale, gewährleisten eine Termination der Transkription und eine Stabilisation des Transkripts. Beispiele für regulatorische Elemente für eukaryotische Wirtszellen sind z.B. der AOX1 oder GAL1 Promotor für Hefen oder der CMV-, der SV40-, der RSV-Promotor (Rous Sarcoma Virus), der CMV-Enhancer, der SV40-Enhancer oder ein Globin-Intron für Säugerzellen oder andere tierische Zellen. In diesem Zusammenhang aus dem Stand der Technik bekannte Expressionsvektoren umfassen u.a. Vektoren wie den Okayama-Berg cDNA Expressionvektor pcDV1 (Pharmacia), pCDM8, pRc/CMV, pcDNA1, pcDNA3 (InVitrogen), pEF-DHFR und pEF-ADA (Raum et al. Cancer Immunol Immunother (2001) 50(3), 141-150) ,pSPORT1 (GIBCO .BRL), sowie pKNOH2 und pLNOK (Norderhaug et al., 1997. J. Immunol. Methods, 204: 77-87).

Der Begriff "Kontrollsequenz" wird verwendet als Beschreibung aller mindestens für eine Expression in einem ausgewählten Wirt notwendigen Sequenzen und kann somit weitere Komponenten umfassen.

Die Erfindung betrifft fernen einen Wirt, transformiert oder transfiziert mit einem oben beschriebenen Vektor.
Dieser Wirt kann ein Prokaryot oder bevorzugt eine eukaryotische Zelle sein. Prokaryotische Wirte umfasst im Zusammenhang mit der Erfindung alle Eubakterien und Archaebakterien, die mit den Nukleinsäuren transformiert werden können. Dies Gruppe umfasst somit Gram-negative wie Gram-positive Bakterien wie z.B. E. coli, S. typhimurium, Serratia marcescens und Bacillus subtilis. Eukaryotische Zellen umfassen u.a. Hefen, höhere Pflanzen, Insekten und, vorzugsweise, Säugerzellen, wie CHO-, COS-7-, NS0-, und Per.C6-Zellen.

Die Erfindung betrifft ferner ein Verfahren zur Herstellung eines Bindungsmoleküls, das von einer oder mehreren der oben beschriebenen Nukleinsäuren kodiert wird, umfassend das Züchten des erfindungsgemäßen Wirts unter geeigneten Bedingungen; und Isolieren des Bindungsmoleküls aus der Kultur.

Der transformierte/transfizierte Wirt kann in einem Fermentor gezüchtet werden. Protokolle für eine Züchtung/Kultivierung unterschiedlicher Wirte sind dem Fachmann bekannt. Diese können ohne weitere erfinderische Tätigkeit bestimmt und optimiert werden. Ebenso kennt der Fachmann Verfahren zur Isolation eines rekombinanten (Poly)peptids wie des erfindungsgemäßen Bindungsmoleküls aus einher Kultur bzw. eines Kulturüberstands. Solche Isolationsverfahren umfassen u.a. Ammoniumsulfat-Präzipitation, Affinitätsaufreinigungen (z.B. mit Hilfe von Chromatographiesäulen), Gelefektrophorese und ähnliches; siehe u.a. Scopes, "Protein Purification", Springer-Verlag, N.Y. (1982) oder Rehm, "Der Experimentator: Proteinbiochemie/Proteomics", Spektrum, Akademischer Verlag. Im wesentliche reine Präparationen von Bindungsmolekülen mit 90 bis 95% Homogenität, vorzugsweise mit 98 bis 99% oder noch höherer Homogenität, sind für pharmazeutische Verwendungen bevorzugt.

In einer weiteren Ausführungsform betrifft die Erfindung ein Bindungsmolekül, kodiert von einer erfindungsgemäßen Nukleinsäure oder mehreren erfindungsgemäßen Nukleinsäuren oder hergestellt mit dem erfindungsgemäßen Verfahren.
Antikörper oder Fragmente oder Derivate eines Antikörpers die einem erfindungsgemäßes Bindungsmolekül entsprechen oder dieses enthalten sind bevorzugt.
Antikörperfragmente im Sinne der Erfindung umfassen Fragmente der vorstehend beschriebenen Antikörper, die sowohl die spezifischen Bindungseigenschaften der definierten variablen Regionen umfassen, als auch über einen Teil der konstanten Region eines IgG1 oder IgG4 Antikörpers verfügen, der eine Kreuzvernetzung ermöglicht.
Derivate von erfindungsgemäßen Antikörpern umfassen, sind jedoch nicht beschränkt auf, markierte Antikörper/Antikörperfragmente, wie auch chemisch modifizierte Antikörper/Antikörperfragmente. Die Markierung/Modifikation der Antikörper/Antikörperfragmente kann posttranslational oder durch chemische/biochemische oder molekularbiologische Modifikation erfolgen. Diese Modifikationen umfassen z.B. eine Modifikation der Glykosylierung (Umana et al., 1999, Nature Biotech. 17: 176-180) und eine PEGylierung (Delgado et al., 1996. J. Drug Target. 3: 321-340).

Vorzugweise ist der erfindungsgemäße Antikörper ein monoklonaler Antikörper.

In einer alternativen Ausführungsform betrifft die Erfindung eine Zusammensetzung, welche die erfindungsgemäße(n) Nukleinsäure(n), einen erfindungsgemäßen Vektor, Wirt, Bindungsmolekül und/oder den Antikörper oder Fragment oder Derivat eines Antikörpers umfasst.

Es ist bevorzugt, das die erfindungsgemäße Zusammensetzung eine pharmazeutische Zusammensetzung ist. Gegebenenfalls kann diese darüberhinaus einen pharmazeutisch verträglichen Träger, einen pharmazeutisch verträglichen Exzipienten oder ein pharmazeutisch verträgliches Verdünnungsmittel enthalten.

Eine erfindungsgemäße pharmazeutische Zusammensetzung umfasst u.a. Arzneimittel und Arzneimittelzubereitungen. Beispiele für besonders geeignete pharmazeutisch/pharmakologisch verträgliche Träger/Exipienten und Verdünnungsmittel sind dem Fachmann bekannt. Diese umfassen gepufferte Kochsalzlösungen, Wasser, Emulsionen wie z.B. ÖI/Wasser-Emulsionen, verschiedene Arten von Detergenzien, sterile Lösungen, etc. Arzneimittel, die solche Träger umfassen, können mittels bekannter konventioneller Methoden formuliert werden. Die erfindungsgemäße pharmazeutische Zusammensetzung kann einem Individuum in einer geeigneten Dosis verabreicht werden. Die Verabreichung kann parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,001 und 500000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 10 ng- und 10 mg-Einheiten pro Tag bzw. pro Applikationsintervall befinden. Wird die Zusammensetzung intravenös verabreicht sollte sich die Dosis in einem Bereich zwischen 1 ng- und 1 mg-Einheiten pro Kilogramm Körpergewicht pro Minute befinden.

Die erfindungsgemäße pharmazeutische Zusammensetzung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäße Zusammensetzung kann außerdem Konservierungsmittel und andere Zusätze umfassen, wie z.B. antimikrobielle Verbindungen, Antioxidantien, Komplexbildner und inerte. Gase, und/oder Substanzen, welche die Löslichkeit fördern, wie beispielsweise Tween. Des weiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein.

Alternativ ist bevorzugt, dass die erfindungsgemäße Zusammensetzung eine diagnostische Zusammensetzung ist.
Es ist ebenso bevorzugt, dass die erfindungsgemäße Zusammensetzung ein Kit ist, der die Nukleinsäure(n), den Vektor, den Wirt, das Bindungsmolekül, und/oder den Antikörper oder Fragment oder Derivat eines Antikörpers in einem oder mehreren Behältern umfasst. Weiter bevorzugt umfasst dieser Kit einen Beipackzettel. Dieser kann eine Beschreibung des Kits, seiner Bestandteile und/oder seiner Verwendung umfassen. Die Beschreibung der Verwendung kann darüber hinaus Dosierungsanweisungen für einen Arzt umfassen.

In einer Ausführungsform betrifft die Erfindung die Verwendung der erfindungsgemäßen Nukleinsäure(n), des erfindungsgemäßen Vektors, des erfindungsgemäßen Wirts, des erfindungsgemäßen Bindungsmoleküls und/öder des erfindungsgemäßen Antikörpers oder Fragments oder Derivats eines erfindungsgemäßen Antikörpers zur Herstellung einer pharmazeutischen Zusammensetzung zur Behandlung von Krankheiten, die mit einer fehler- oder mangelhaften Co-stimulierbarkeit, Funktionalität oder Anzahl von T-Zellen einhergehen, von entzündlichen Krankheiten, und/oder von Autoimmunerkrankungen.

Alternativ dazu betrifft die Erfindung ein Verfahren zur Behandlung von Krankheiten, die mit einer fehlerhaften Co-stimulierbarkeit, Funktionalität oder Anzahl von T-Zellen einhergehen, von entzündlichen Krankheiten, und/oder von Autoimmunerkrankungen, umfassend die Verabreichung der erfindungsgemäßen Nukleinsäure(n), des erfindungsgemäßen Vektors, des erfindungsgemäßen Wirts, des erfindungsgemäßen Bindungsmoleküls, und/oder des erfindungsgemäßen Antikörpers oder Fragments oder Derivats eines erfindungsgemäßen Antikörpers.

Erfindungsgemäße Verwendungen und Verfahren sind dann bevorzugt, wenn die Krankheiten, die mit einer fehlerhaften Co-stimulierbarkeit, Funktionalität oder Anzahl von T-Zellen einhergehen; ausgewählt sind aus der Gruppe bestehend aus chronisch lymphozytäre Leukämie des B-Zell-Typs (B-CLL), akute lymphoblastische Leukämie (ALL), T-Lymphopenie, soliden Tumoren, HIV-Infektion und HTLV-Infektion. Die soliden Tumore sind vorzugsweise ausgewählt aus Nierenkarzinomen, Lungenkarzinomen und Melanomen.

Alternativ bevorzugt sind Verwendungen und Verfahren, wobei die entzündlichen und Autoimmunerkrankungen ausgewählt sind aus der Gruppe bestehend aus rheumatoider Arthritis (RA), Typ I Diabetes mellitus, multipler Sklerose, Psoriasis, Guillain-Barre-Syndrom, Morbus Crohn, und Erkrankungen, die auf unerwünschte T-Zellaktivierungsreaktionen zurückzuführen sind wie "graft-versus-host disease" (GvHD) oder "host-versus-graft disease" (HvGD). Bei den HvGDs ist die Abstoßung solider Organtransplantate, wie Leber, Lunge, Herz und Nieren besonders ins Auge gefasst.

In einer weiteren alternativen Ausführungsform betrifft die Erfindung die Verwendung der erfindungsgemäßen Nukleinsäure(n), des erfindungsgemäßen Vektors, des erfindungsgemäßen Wirts, des erfindungsgemäßen Bindungsmolekül und/oder des erfindungsgemäßen Antikörper oder Fragment oder Derivat eines Antikörpers zur Herstellung einer diagnostischen Zusammensetzung zur in-vitro Analyse der Responsivität eines Patienten für eine Therapie mit einer pharmazeutischen Zusammensetzung.

Ein mit dieser diagnostischen Zusammensetzung ausführbare in-vitro Analyse umfasst beispielsweise folgende Schritte:
(a) Isolation von Blutzellen aus einer Blutprobe (z.B. PBMCs (peripheren molonukleären Zellen aus Blut) und/oder Lymphozyten);
(b) Inkubation der Blutzellen mit einem erfindungsgemäßen Antikörper und ggf. Zugabe eines exogenen Kreuzvemetzungsreagenz;
(c) Detektion einer Antikörper-abhängigen Stimulation von T-Zellen in der Probe. Wird eine Antikörper-abhängigen Stimulation von T-Zellen in der Probe detektiert, so ist der Patient, von dem die Probe stammt, responsiv für eine Therapie mit einer erfindungsgemäßen pharmazeutischen Zusammensetzung.

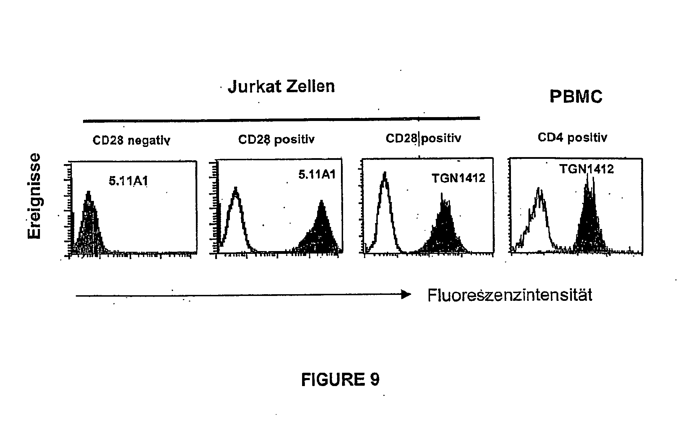

Die Figuren zeigen:
**Figuren 1-8****:** zeigen die Nukleotid-bzw. Aminosäuresequenzen der leichten und schweren Ketten von TGN1412 und TGN1112.
**Figur 9****:** Bindungsspezifität der Antikörper TGN1412 und 5.11A1 für CD28 auf transfizierten Jurkat Zellen und auf primären menschlichen T-Zellen.
   Für Immunfluoreszenfärbungen und Durchflusszytometrie wurden je 1-2 x 10⁶ Zellen/ml in FACS Puffer (PBS, BSA, Na-Azid) mit sättigenden Mengen der Antikörper 5.11A1, TGN1412 irrelevanten, lsotyp-spezifischen Kontrollantikörpem für 40 Minuten bei 4°C inkubiert. Die Färbungen wurden gewaschen, um überschüssigen Antikörper zu entfernen, und vor der Analyse mit sättigenden Mengen an PE-konjugiertem anti-Maus IgG (für 5,11A1) bzw. PE-konjugiertem anti-human IgG (für TGN1412) für 15 Minuten inkubiert. Die Zellen wurden anschließend mit anti-CD4 Antikörper gegengefärbt. Die durchflusszytometrische Analyse erfolgte an einem FACSCalibur™ mit Hilfe der Cell Quest™ Software (Becton Dickinson). Fluoreszenzsignale und Side-scatter (ssc) Signale wurden logarithmisch, Forward-scatter (fsc) Signale linear aufgenommen. Histogramme zeigen durchweg Signale von Zellen, die in einem empirischen "Life-Gate", definiert durch ssc und fsc, liegen. Sie zeigen die Bindung von 5.11A1 oder TGN1412 (ausgefüllte Kurven) im Vergleich zu Isotyp-Kontroll-Antikörpern (offene Kurven) an Jurkat-Zellen, die kein CD28 exprimieren (linke Graphik) bzw. mit CD28 transfiziert wurden (mittlere Graphiken), sowie an CD4 positive periphere Blut-Mononukleäre Zellen (PBMC, rechte Graphik).
**Figur 10****:** Zellgewinnung und Proliferationstests.
   PBMC wurden durch Ficoll-Dichtezentrifugation aus frisch gewonnenen peripherem Blut bzw. aus Buffy Coats isoliert, gewaschen und in einer Konzentration von 2 x 10⁵ Zellen pro 200 µl Napf in 96-Napf Rundbodenplatten in Kultur genommen. Entsprechend den Beschriftungen wurden sie mit löslichem TGN1112, TGN1412, 5.11A1 oder Isotyp-Kontrollantikörpern in einer Konzentration von 1 µg/ml (A) oder entsprechend der Beschriftung an der Abszisse (B) kultiviert. Nach 48 Stunden wurde die relative Zellproliferationsrate -dargestellt als mean cpm ± SD = Mittelwert counts-per-minute ± Standardabweichung- mittels [*methyl*-³H] Thymidin-Einbau über einen Zeitraum von 18 Stunden und anschließender Flüssig-Szintillationsmessung bestimmt. Die Ergebnisse sind repräsentativ für multiple Bestimmungen mit diversen Spendern.

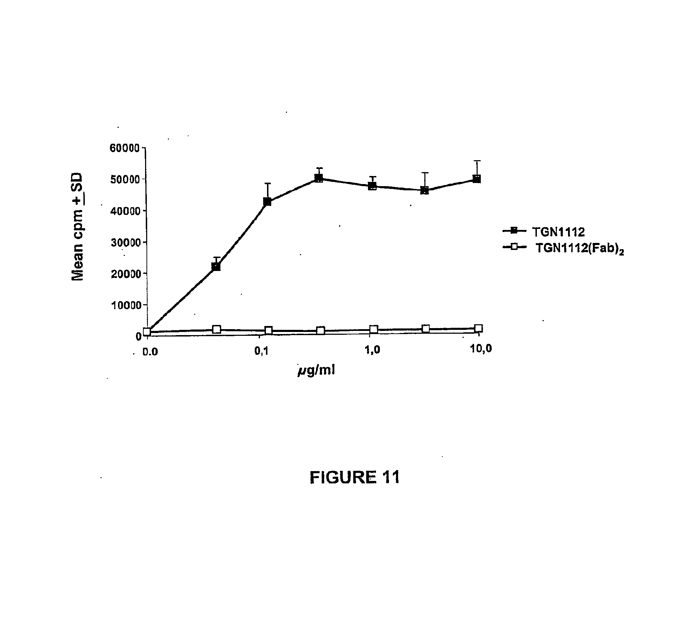

**Figur 11****:** Zellgewinnung, PBMC-Stimulation und Darstellung waren wie für Figur 10 beschriebenen. Die Stimulation von TGN1112 wurde mit der von TGN1112(Fab)₂-Fragmenten verglichen, deren biochemische Qualität und spezifische Bindung an CD28 Transfektanten zuvor gezeigt worden war.

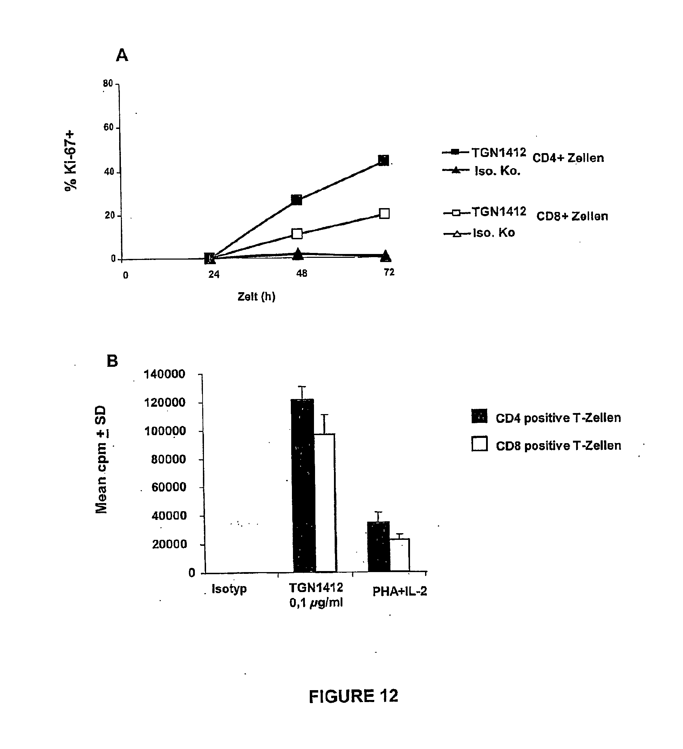

**Figur 12****:** Stimulation von CD4 und CD8 T-Zellen durch TGN1412.
   (A) PBMC (Gewinnung und Kulturbedingungen siehe Figur 10) wurden mit TGN1412 (1 µg/ml) bzw. einem Isotyp-Kontroll-Antikörper für die angegebene Zeiträume stimuliert. Der Zellzyklus-Status für CD4 bzw. CD8 Zellen wurde durch intrazelluläre Färbung mittels FITC-markiertem anti- Ki-67 Antikörper bestimmt. Dazu wurden Zellen mit anti-human CD4 PE bzw. anti-CD8 PE Antikörpern für 15 Minuten inkubiert, gewaschen und in Cytofix/cytoperm™ Puffer (Becton Dickinson) für 20 Minuten bei 4°C inkubiert. Nach Waschen mit Perm/Wash™ Puffer (Becton Dickinson) wurden die Zellen für weitere 30 Minuten bei 4°C mit anti-Ki-67-FITC gefärbt, gewaschen und durchflusszytometrisch wie unter Figur 9 beschrieben gemessen.
   (B) CD4+ bzw. CD8+ T-Zellen wurden aus einer PBMC Suspension mit Hilfe geeigneter T-Zell-Isolierungskits und anschließender AutoMACS™-Separation (beides Miltenyi) gewonnen. Die durchfluszytometrisch überprüfte Reinheit betrug 93-98% für CD4+ und 81-95% für CD8+ T-Zellen. 96 Napf-Flachbodenplatten wurden mit anti-human-Ig (40 µg/ml in PBS) für 3 h bei Raumtemperatur beschichtet und anschließend gewaschen. Die Zellen wurden anschließend in einer Dichte von 1 x 10⁵ /Napf zusammen mit TGN1412 oder einem Referenzantikörper kultiviert. Als Positivkontrolle für Zellproliferation diente der Zusatz von PHA (5 µg/ml) und IL-2 (200 U/ml). Die Proliferation wurde nach 48 Stunden wie für Figur 10 beschrieben bestimmt und stellt ein repräsentatives Ergebnis für verschiedene Donoren dar.

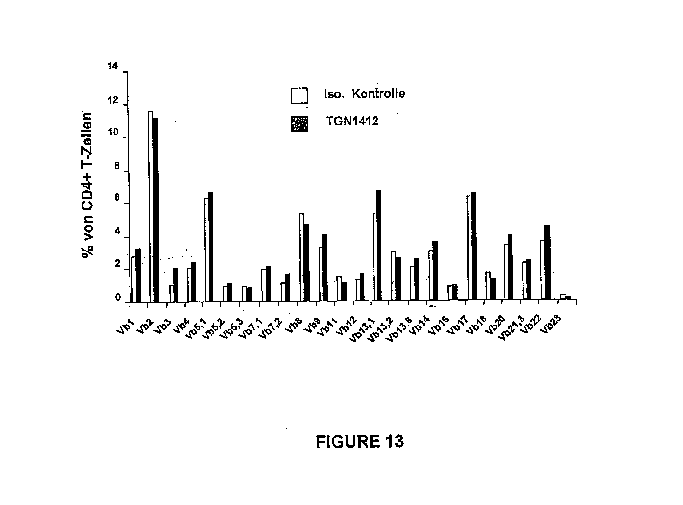

**Figur 13****:** Analyse des TCR Repertoires TGN1412-stimulierter PBMC. Die TCR V[beta] Repertoire-Analyse von PBMC, die 96 Stunden mit TGN1412 (1µg/ml) bzw. einem 1gG4 Kontrollantikörper kultiviert wurden, erfolgte mit Hilfe des IOTest® Beta Mark TCR V[beta] Repertoire. Kit™ (Beckman Coulter) nach Herstellerangaben. Proben wurden mit anti-CD3 und anti-CD4 Antikörpern wie für Figur 9 beschrieben gegengefärbt. Dargestellt ist ein repräsentatives Ergebnis dreier Bestimmungen, bei dem das Analysefenster auf CD3+ CD4+ Zellen gesetzt wurde.

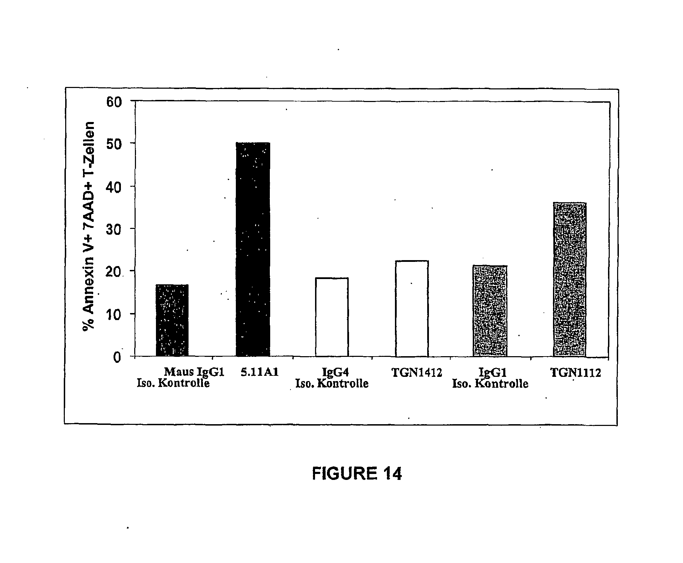

**Figur 14****:** Messung der anti-CD28 vermittelten T-Zell Apoptose.
   PBMC (2 x 10⁵/ml) wurden mit löslichem 5.11A1, TGN1412, TGN1112 oder lsotypkontrollantikörper (1 µg(ml) für 72 Stunden inkubiert. Anschließend wurden die Zellen für 15 Minuten mit anti-CD3-APC gefärbt und mit FACS Puffer gewaschen. PBMC wurden in Annexin V Binding Buffer (BD Pharmingen) resuspendiert und mit anti-Annexin V und 7AAD für 15 Minuten inkubiert. Die Apoptose im Sinne von T-Zellen (CD3+ Fenster), welche Annexin V positiv und 7AAD permeabel waren, wurden mittels Durchflusszytometrie innerhalb 1 Stunde bestimmt.
**Figur 15****:** Induktion des pro-apoptischen Rezeptors CD95 durch anti-CD28 Antikörper.
   Der Versuchsaufbäu entspricht dem in Figur 14 beschriebenen. Anstelle der Annexin V / 7AAD Färbung wurden PBMC mit anti-CD3 FITC und CD95-PE gefärbt und es wurde die Expression von CD95 auf CD3+ T-Zellen mittels Durchflusszytometrie bestimmt. Gezeigt ist ein beispielhaftes Ergebnis für zwei verschiedene Donoren.
**Figur 16****:** Induktion von Antikörper-abhängiger zellulärer Zytotoxizität, ADCC. Die ADCC diverser anti-CD28 Antikörper wurde durchflusszytometrisch bestimmt. Dazu wurden 5x10⁴ Calcein-AM markierte CD28+ Jurkat Zellen mit frisch isolierten PBMC in Anwesenheit von 6µg/ml der angegebenen Antikörper bei den dargestellten Effektor:Zielzellen-Verhältnissen (E:T) für 3,5 Stunden ko-kultiviert. Die Zellen wurden mit 0.5µg/ml Propidium-lodid in FACS Puffer aufgenommen und durchflusszytometrisch vermessen. Das Verhältnis von Calcein negativen oder Calcein schwach positiven Zellen / Propidium-lodid positiven Zellen (x100) zu gesamt-Calcein positiven Zellen wurde als Prozentanteil an Zytotoxizität bestimmt. Gezeigt ist ein repräsentatives Ergebnis von Bestimmungen mit Zellen verschiedener Donoren.
**Figur 17****:** CDRs der erfindungsgemäßen Bindungsmoleküle nach Kabat-System und nach dem AbM/Kabat-Kombi-System.

Die Beispiele erläutern die Erfindung. Die Beispiele sollen nicht als Limitierung der Erfindung angesehen werden. Die Beispiele sind lediglich zur Illustration der Erfindung beigefügt und die Erfindung wird lediglich durch die Ansprüche begrenzt.

### Beispiel 1: Bindungseigenschaften der erfindungsgemäßen Antikörper an CD28, CD16b, CD32 und CD64

Es wurde untersucht, ob bei dem Humanisierungsprozess die Bindung an das CD28 Molekül des Menschen erhalten blieb. Wie in Fig.9 beispielhaft für TGN1412 gezeigt, war dies für beide Isotypen der Fall.

Anschließend wurden die Bindungseigenschaften von TGN1412 und TGN112 an CD16b, CD32 und CD64 ermittelt. Die Ergebnisse sind in Tabelle 1 zusammengefasst. Es zeigte sich erwartungsgemäß, dass TGN1112 mit gut nachweisbarer Affinität an CD16 und CD64, nicht jedoch an CD32 bindet, während TGN1412 nur schwach und ausschließlich mit CD64 interagierte.

**Tabelle 1: Bindungseigenschaften vonTGN1412 und TGN1112 on humane Fc gamma Rezeptoren**

| FcγR | TGN1112 | TGN1412 |
|---|---|---|
| CD16b | + | - |
| CD32 | - | - |
| CD64 | ++ | + |

| | | |
|---|---|---|
| Legende: - nicht detektierbar + mittlere Bindung ++ starke Bindung | | |

Tabelle 1: Die Bindung von TGN1412 und TGN1112 an FcγRIIIb (CD16b) wurde mittels ELISA gemessen wobei humanes, rekombinantes CD16b (R&D Sytems) als "Capture-Antigen" diente. Die Interaktion zwischen CD16b und TGN1112 wurde mittels HRP-konjugiertem anti-human light chain kappa Ig nachgewiesen. Zum Nachweis der Bindung von TGN1112 bzw. TGN1412 an CD32 und CD64 wurden humane, CD28 negative Zelllinien, welche konstitutiv CD32 und/oder CD64 exprimieren, mit TGN1112 oder TGN1412 für 40 Minuten inkubiert, gewaschen und mit PE- konjugiertem anti-human IgG Antikörper, der die kappa Kette erkennt, gegengefärbt. Zellen wurden gewaschen und die Bindung von TGN1112 und TGN1412 an FcyR wurde mittels Durchflusszytometrie detektiert. Die Spezifität für CD64 wurde bei Zellinien, welche sowohl CD32 als auch CD64 exprimieren, durch Kompetitionsexperimente mit blockierendem anti-CD64 Antikörper ermittelt.

### Beispiel 2: T-Zell-Stimulation durch die erfindungsgemäßen Antikörper

Nachfolgend sind experimentelle Befunde erläutert, die zeigen, dass überraschender Weise sowohl TGN1112 als auch TGN1412 ohne künstliche Quervernetzung in der Lage sind, ex vivo humane T-Lymphozyten zu aktivieren. Aufgrund der Tatsache, dass TGN1412 nur schwach an den Fc-Rezeptor CD64 bindet, war dies insbesondere für TGN1412 nicht zu erwarten.

Es wurde zunächst untersucht, ob die T-Zell-aktivierenden Eigenschaften der humanisierten Antikörper gegenüber dem Ausgangsantikörper 5.11A1 erhalten blieben. Das überraschende Ergebnis ist in Fig.10 beispielhaft dargestellt. In Fig.10A ist gezeigt, dass sowohl TGN1112 als auch TGN1412 in löslicher Form in der Lage sind, in Zellsuspension periphere Blut-mononukleäre Zellen (PBMC) effizient zur Proliferation anzuregen, In Fig. 10B ist gezeigt, dass die Induktion der Proliferation von T-Zellen durch TGN1412 ähnlich stark erfolgt wie die Induktion der Proliferation durch den Ausgangsantikörper 5.11A1, dass jedoch konventionelle, d.h. nichtsuperagonistische anti-CD28 Antikörper wie beispielsweise die Antikörper 28.2 oder 152-2E10 dazu nicht in der Lage sind.

### Beispiel 3: Relevanz der Fc-Domäne für die stimulatorischen Eigenschaften

Anschließend wurde untersucht, ob die Fc-Domäne für die stimulatorischen Eigenschaften gegenüber menschlichen T-Zellen in einer Kultur von PBMC notwendig ist. Dazu wurden von TGN1112 F(ab)₂ Fragmente hergestellt und diese in Zellkultur mit intaktem Antikörper hinsichtlich der Aktivierung von PBMC verglichen. Das Ergebnis ist in Fig. 11 dargestellt. Es zeigt sich, dass F(ab)₂ Fragmente von TGN1112 keinerlei Proliferation induzierten, während der intakte Antikörper dosisabhängig eine starke Proliferation der PBMC auslöste.

### Beispiel 4: T-Zellen sind die Zielzellen der stimulatorischen Aktivität der erfindungsgemäßen Antikörper

Um zu zeigen, dass es sich bei den durch TGN1412-Stimulation proliferierenden Zellen in der PBMC Kultur um T-Zellen handelt, wurde die Analyse des Zellzyklusmarkers Ki-67 mit der Analyse der Oberflächenmarker CD4 und CD8 verknüpft. Die Expression von CD4 bzw. CD8 kennzeichnet die beiden Hauptgruppen von T-Zellen, T-Hefferzellen (CD4 exprimierend) bzw. zytotoxische T-Zellen (CD8 exprimierend). In Fig.12A ist gezeigt, dass löslicher TGN1412 die Proliferation beider Untergruppen in einer PBMC Suspension deutlich anregt. In Fig.12B ist gezeigt, dass auch gereinigte CD4 bzw. CD8 T-Lymphozyten durch TGN1412 Stimulation deutlich zur Proliferation angeregt werden, allerdings ist in diesem System die Anwesenheit eines quervernetzenden Agens notwendig.

In Fig. 13 ist gezeigt, dass durch die TGN1412-vermittelte Expansion von CD4 T-Zellen in Zellkultur das Repertoire der TCRVB Expression erhalten bleibt. Dies ist dahingehend ein wichtiger Befund, dass es in der angestrebten Immuntherapie von Nutzen ist, die natürlich vorkommende Vielfalt des TCR Repertoire zu konservieren, um Immuntoleranz zu erhalten und Reaktivität gegenüber einem breiten Spektrum von möglichen Erregern zu gewährleisten.

Zusammenfassend zeigen diese Ergebnisse überraschender Weise, dass sowohl TGN1412 als auch TGN1112 in löslicher Form in der Lage sind, in Zellkultur menschliche T-Zellen zu stimulieren.

### Beispiel 5: Hohe stimulatorische bei geringer pro-apoptotischer Wirkung der erfindungsgemäßen Antikörper

Anschließend wurde die T-Zell-Aktivierung, welche durch TGN1412, TGN1112 und den Ausgangsantikörper 5.11A1 vermittelt wurde, hinsichtlich der Induktion von programmiertem Zelltod (Apoptose) genauer untersucht. Repräsentative Ergebnisse sind in Fig.14 und Fig.15 zusammengefasst.

In Fig.14 ist dargestellt, dass der Anteil apoptotischer T-Zellen innerhalb einer PBMC Kultur, welche mit den anti-CD28 Antikörpern 5.11A1, TGN1412 und TGN1112 in löslicher Form stimuliert wurden, bei den 5.11A1 stimulierten Kulturen am häufigsten, bei den TGN1112 stimulierten Kulturen intermediär und bei den TGN1412 stimulierten Kulturen am geringsten ausgeprägt war. Im Einklang mit diesen Ergebnissen zeigt Fig.15, dass die Expression des Apoptose-auslösenden Rezeptors CD95 auf T-Zellen, die mit 5.11A1 stimuliert waren, am häufigsten war, während TGN1112 stimulierte Zellen eine mittlere und TGN1412 stimulierte T-Zellen ein geringe Häufigkeit an CD95 positiven Zellen aufwiesen.

### Literatur

Delgado et al., 1996. J. Drug Target. 3: 321-340
Evans et al., Nature Immunol., 2005, 6: 271-279
Ewert et al., 2004. Methods, 34: 184-199
Hwang et al., Methods, 2005, 36:3-10
Isaacs et al., Clin. Exp. Immunol., 106: 427-433
Johnson, G. and Wu, T. T. 2000, Nucleic Acids Research
Jones et al. 1986. Nature 321: 522-525
Kabat, E. A. et al. 1991; Sequences of Proteins of Immunological Interest, Fifth Edition. NIH Publication No. 91-3242
Lin et al., Eur. J. Immunol., 2003, 33:626-638
Luhder et al., J. Exp. Med., 2003, 197: 955-966
Norderhaug et al., 1997. J. Immunol. Methods, 204: 77-87
Schmidt et al., J. Neuroimmunol., 2003, 140: 143-152
Schwartz, Nature Immunol., 2005, 6: 327-330
Tacke et al., Eur. J. lmmunol., 1997, 27:239-247
Umana et al., 1999, Nature Biotech. 17: 176-180
Woof et al., Nature Reviews Immunol., 2004, 1-11

## Patentansprüche

1. Nukleinsäure, kodierend ein Bindungsmolekül, das spezifisch ein menschliches CD28-Molekül bindet, umfassend
(a) eine Nukleinsäuresequenz kodierend eine V_{H}-Region und eine Nukleinsäuresequenz kodierend eine V_{L}-Region umfassend CDRs in einem humanen Immunglobulin-Gerüst, wobei
(i) die CDRs der V_{H}-Region (CDR-H) die Aminosäuresequenzen der SEQ ID NO: 2 oder 18 (CDR-H3), 4 oder 20 (CDR-H2) und 6 oder 22 (CDR-H1) umfassen oder kodiert werden durch die Nukleinsäuresequenzen der SEQ ID NR: 1 oder 17 (CDR-H3), 3 oder 19 (CDR-H2) und 5 oder 21 (CDR-H1); und
(ii) die CDRs der V_{L}-Region (CDR-L) die Aminosäuresequenzen der SEQ ID NO: 8 oder 24 (CDR-L3), 10 oder 26 (CDR-L2) und 12 oder 28 (CDR-L1) umfassen oder kodiert werden durch die Nukleinsäuresequenzen der SEQ ID NR: 7 oder 23 (CDR-L3), 9 oder 25 (CDR-L2) und 11 oder 27 (CDR-L1); und
(b) eine Nukleinsäuresequenz, die die konstante Region eines humanem IgGI- oder IgG4-Antikörpers kodiert.

2. Nukleinsäure nach Anspruch 1, umfassend eine Nukleinsäuresequenz kodierend eine V_{H}-Region und eine Nukleinsäuresequenz kodierend eine V_{L}- Region, wobei
(i) die Nukleinsäuresequenz der V_{H}-Region SEQ ID NR: 33 oder 37 umfasst
und/oder ein (Poly)peptid kodiert, das die Aminosäuresequenz SEQ ID NR: 34 oder 38 umfasst; und
(ii) die Nukleinsäuresequenz der V_{L}-Region SEQ ID NR: 35 oder 39 umfasst
und/oder ein (Poly)peptid kodiert, das die Aminosäuresequenz SEQ ID NR: 36 oder 40 umfasst.

3. Nukleinsäure nach Anspruch 1 oder 2, umfassend eine Nukleinsäuresequenz, wobei die Nukleinsäuresequenz
(i) SEQ ID NR: 13, 29, 41 oder 45 ist; und/oder
(ii) ein (Poly)peptid mit der Aminosäuresequenz SEQ ID NR: 14, 30, 42 oder 46 kodiert,
insbesondere umfassend eine Nukleinsäuresequenz, wobei die Nukleinsäuresequenz
(i) SEQ ID NR: 15, 31, 43 oder 47 ist; und/oder
(ii) ein (Poly)peptid mit der Aminosäuresequenz SEQ ID NR: 16, 32, 44 oder 48 kodiert,
optional darüber hinaus umfassend eine Nukleinsäuresequenz kodierend für ein Markierungselement oder Tag.

4. Vektor, umfassend die Nukleinsäure nach einem der Ansprüche 1 bis 3, oder Wirt, transformiert oder transfiziert mit einem solchen Vektor.

5. Verfahren zur Herstellung eines Bindungsmoleküls, das von einer Nukleinsäure nach einem der Ansprüche 1 bis 3 kodiert wird, umfassend Züchten des Wirts nach Anspruch 4 unter geeigneten Bedingungen; und Isolieren des Bindungsmoleküls aus der Kultur.

6. Bindungsmolekül, kodiert von einer Nukleinsäure nach einem der Ansprüche 1 bis 3 oder hergestellt mit dem Verfahren nach Anspruch 5.

7. Antikörper, insbesondere monoklonaler Antikörper, oder Fragment oder Derivat eines Antikörpers, insbesondere eines monoklonalen Antikörpers, umfassend zumindest ein Bindungsmolekül nach Anspruch 6.

8. Zusammensetzung, umfassend die Nukleinsäure nach einem der Ansprüche 1 bis 3, den Vektor nach Anspruch 4, den Wirt nach Anspruch 4, das Bindungsmolekül nach Anspruch 6, und/oder den Antikörper oder Fragment oder Derivat eines Antikörpers nach Anspruch 7,
wobei die Zusammensetzung vorzugsweise eine pharmazeutische Zusammensetzung ist und gegebenenfalls darüberhinaus einen pharmazeutisch verträglichen Träger, einen pharmazeutisch verträglichen Exzipienten oder ein pharmazeutisch verträgliches Verdünnungsmittel enthält oder
wobei die Zusammensetzung eine diagnostische Zusammensetzung ist.

9. Zusammensetzung nach Anspruch 8, die ein Kit ist, der die Nukleinsäure, den Vektor, den Wirt, das Bindungsmolekül, und/oder den Antikörper oder Fragment oder Derivat eines Antikörpers in einem oder mehreren Behältern umfasst.

10. Verwendung der Nukleinsäure nach einem der Ansprüche 1 bis 3, den Vektor nach Anspruch 4, den Wirt nach Anspruch 4, das Bindungsmolekül nach Anspruch 6, und/oder den Antikörper oder Fragment oder Derivat eines Antikörpers nach Anspruch 7 zur Herstellung einer pharmazeutischen Zusammensetzung zur Behandlung von Krankheiten, die mit einer fehler- oder mangelhaften Co-stimulierbarkeit, Funktionalität oder Anzahl von T-Zellen einhergehen, von entzündlichen Krankheiten, und/oder von Autoimmunerkrankungen.

11. Verfahren zur Behandlung von Krankheiten, die mit einer fehlerhaften Co- stimulierbarkeit, Funktionalität oder Anzahl von T-Zellen einhergehen, von entzündlichen Krankheiten, und/oder von Autoimmunerkrankungen, umfassend die Verabreichung der Nukleinsäure nach einem der Ansprüche 1 bis 3, den Vektor nach Anspruch 4, den Wirt nach Anspruch 4, das Bindungsmolekül nach Anspruch 6, und/oder den Antikörper oder Fragment oder Derivat eines Antikörpers nach Anspruch 7.

12. Verwendung nach Anspruch 10 oder Verfahren nach Anspruch 11, wobei die Krankheiten, die mit einer fehlerhaften Co-stimulierbarkeit, Funktionalität oder Anzahl von T-Zellen einhergehen, ausgewählt sind aus der Gruppe bestehend aus chronisch lymphozytäre Leukämie des B-Zell-Typs (B-CLL), akute lymphoblastische Leukämie (ALL), T-Lymphopenie, soliden Tumoren, HIV-Infektion und HTLV-Infektion.

13. Verwendung nach Anspruch 10 oder Verfahren nach Anspruch 11, wobei die entzündlichen und Autoimmunerkrankungen ausgewählt sind aus der Gruppe bestehend aus rheumatoider Arthritis (RA), Typ I Diabetes mellitus, multipler Sklerose, Psoriasis, Guillain-Barre-Syndrom, Morbus Crohn, und Erkrankungen, die auf unerwünschte T-Zellaktivierungsreaktionen zurückzuführen sind wie "graft-versus-host disease" (GvHD) oder "host-versus-graft disease" (HvGD).

14. Verwendung der Nukleinsäure nach einem der Ansprüche 1 bis 3, den Vektor nach Anspruch 4, den Wirt nach Anspruch 4, das Bindungsmolekül nach Anspruch 6, und/oder den Antikörper oder Fragment oder Derivat eines Antikörpers nach Anspruch 7 zur Herstellung einer diagnostischen Zusammensetzung zur in-vitro Analyse der Responsivität eines Patienten für eine Therapie mit einer pharmazeutischen Zusammensetzung nach Anspruch 8 in der Ausführungsform einer pharmazeutischen Zusammensetzung.
